# EUROPEAN PATENT SPECIFICATION

(11) **EP 1 549 313 B1**
(45) Date of publication and mention of the grant of the patent: **27.06.2012**
(21) Application number: 03784906.4
(22) Date of filing: 05.08.2003
(51) Int. Cl.: C07D 471/04, C07D 487/04, A61K 31/496, A61K 45/06, A61P 31/18

(54) **COMPOSITION AND ANTIVIRAL ACTIVITY OF SUBSTITUTED AZAINDOLEOXOACETIC PIPERAZINE DERIVATIVES**
ZUSAMMENSETZUNG UND ANTIVIRALE AKTIVITÄT VON SUBSTITUIERTEN AZAINDOLOXOESSIGSÄURE-PIPERAZIN-DERIVATEN
COMPOSITION ET ACTIVITE ANTIVIRALE DE DERIVES DE PIPERAZINE AZAINDOLEOXOACETIQUE SUBSTITUES

(30) Priority: 07.08.2002 US 214982
(43) Date of publication of application: 06.07.2005
(73) Proprietor: Bristol-Myers Squibb Company, Princeton NJ 08543-4000 (US)
(72) Inventor: WANG, Tao, Farmington, CT 06032 (US); ZHANG, Zhongxing, Madison, CT 06443 (US); MEANWELL, Nicholas, A., East Hampton, CT 06424 (US); KADOW, John, F., Wallingford, CT 06492 (US); YIN, Zhiwei, Glastonbury, CT 06033 (US); XUE, Qiufen, May, Newbury Park, CA 91320 (US); REGUEIRO-REN, Alicia, Middletown, CT 06457 (US); MATISKELLA, John, D., Wallingford, CT 06492 (US); UEDA, Yasutsugu, Clinton, CT 06413 (US)
(74) Representative: Reitstötter - Kinzebach
(86) International application number: PCT/US2003/024415
(87) International publication number: WO 2004/014380

(56) References cited:
- WO-A1-02/062423
- WO-A2-03/072028
- US-A1- 2002 061 892

## Description

### BACKGROUND OF THE INVENTION

### Field of the Invention

This invention provides compounds of the present claims 1 and 2 having drug and bio-affecting properties, their pharmaceutical compositions and their use in antiviral activity. More particularly, the present invention relates to compounds of the present claims 1 and 2 useful for the treatment of HIV and AIDS.

### Background Art

HIV-1 (human immunodeficiency virus -1) infection remains a major medical problem, with an estimated 42 million people infected worldwide at the end of 2002. The number of cases of HIV and AIDS (acquired immunodeficiency syndrome) has risen rapidly. In 2002, ∼5.0 million new infections were reported, and 3.1 million people died from AIDS. Currently available drugs for the treatment of HIV include nine nucleoside reverse transcriptase (RT) inhibitors or approved single pill combinations(zidovudine or AZT (or Retrovir^{®}), didanosine (or Videx^{®}), stavudine (or Zerit^{®}), lamivudine (or 3TC or Epivir^{®}), zalcitabine (or DDC or Hivid^{®}), abacavir succinate (or Ziagen^{®}), Tenofovir disoproxil fumarate salt (or Viread^{®}), Combivir^{®} (contains -3TC plus AZT), Trizivir^{®} (contains abacavir, lamivudine, and zidovudine); three non-nucleoside reverse transcriptase inhibitors: nevirapine (or Viramune^{®}), delavirdine (or Rescriptor^{®}) and efavirenz (or Sustiva^{®}), and eight peptidomimetic protease inhibitors or approved formulations: saquinavir, indinavir, ritonavir, nelfinavir, amprenavir, lopinavir, Kaletra^{®} (lopinavir and Ritonavir), and Atazanavir (Reyataz^{®}). Each of these drugs can only transiently restrain viral replication if used alone. However, when used in combination, these drugs have a profound effect on viremia and disease progression. In fact, significant reductions in death rates among AIDS patients have been recently documented as a consequence of the widespread application of combination therapy. However, despite these impressive results, 30 to 50% of patients ultimately fail combination drug therapies. Insufficient drug potency, non-compliance, restricted tissue penetration and drug-specific limitations within certain cell types (e.g. most nucleoside analogs cannot be phosphorylated in resting cells) may account for the incomplete suppression of sensitive viruses. Furthermore, the high replication rate and rapid turnover of HIV-1 combined with the frequent incorporation of mutations, leads to the appearance of drug-resistant variants and treatment failures when sub-optimal drug concentrations are present (Larder and Kemp; Gulick; Kuritzkes; Morris-Jones *et al*; Schinazi *et al*; Vacca and Condra; Flexner; Berkhout and Ren *et al*; (Ref. 6-14)). Therefore, novel anti-HIV agents exhibiting distinct resistance patterns, and favorable pharmacokinetic as well as safety profiles are needed to provide more treatment options.

Currently marketed HIV-1 drugs are dominated by either nucleoside reverse transcriptase inhibitors or peptidomimetic protease inhibitors. Non-nucleoside reverse transcriptase inhibitors (NNRTIs) have recently gained an increasingly important role in the therapy of HIV infections (Pedersen & Pedersen, Ref 15). At least 30 different classes of NNRTI have been described in the literature (De Clercq, Ref. 16) and several NNRTIs have been evaluated in clinical trials. Dipyridodiazepinone (nevirapine), benzoxazinone (efavirenz) and bis(heteroaryl) piperazine derivatives (delavirdine) have been approved for clinical use. However, the major drawback to the development and application of NNRTIs is the propensity for rapid emergence of drug resistant strains, both in tissue cell culture and in treated individuals, particularly those subject to monotherapy. As a consequence, there is considerable interest in the identification of NNRTIs less prone to the development of resistance (Pedersen & Pedersen, Ref 15).

Several indole derivatives including indole-3-sulfones, piperazino indoles, pyrazino indoles, and 5H-indolo[3,2-b][1,5]benzothiazepine derivatives have been reported as HIV-1 reverse transciptase inhibitors (Greenlee et al, Ref. 1; Williams et al, Ref. 2; Romero et al, Ref. 3; Font et al, Ref. 17; Romero et al, Ref. 18; Young et al, Ref. 19; Genin et al, Ref. 20; Silvestri et al, Ref. 21). Indole 2-carboxamides have also been described as inhibitors of cell adhesion and HIV infection (Boschelli et al, US 5,424,329, Rev. 4). Finally, 3-substituted indole natural products (Semicochliodinol A and B, didemethylasterriquinone and isocochliodinol) were disclosed as inhibitors of HIV-1 protease (Fredenhagen et al, Ref. 22). Other indole derivatives exhibiting antiviral activity useful for treating HIV are disclosed in PCT WO 00/76521 (Ref. 93). Also, indole derivatives are disclosed in PCT WO 00/71535 (Ref. 94).

Structurally related aza-indole amide derivatives have been disclosed previously (Kato et al, Ref. 23; Levacher et al, Ref. 24; Dompe Spa, WO-09504742, Ref. 5(a); SmithKline Beecham PLC, WO-09611929, Ref. 5(b); Schering Corp., US-05023265, Ref. 5(c)). However, these structures differ from those claimed herein in that they are aza-indole mono-amide rather than unsymmetrical aza-indole piperazine diamide derivatives, and there is no mention of the use of these compounds for treating viral infections, particularly HIV. Other azaindoles have been also disclosed by Wang et al, Ref. 95. Indole and azaindole piperazine containing derivatives have been disclosed in four different PCT and issued U.S. patent applications (Reference 93-95, 106). US 2002/0061892 discloses 1H-Pyrrolo[2,3-c]pyridine compounds for the treatment of HIV infections and AIDS. Compounds can additionally comprise an antiviral effective amount of an AIDS treatment agent selected from a group consisting of an AIDS antiviral agent, an anti-infective agent, an immunomodulator, and HIV entry inhibitors. The present compounds differ from the compounds in US 2002/0061892 in 1,2,3-triazole or 3-methyl-1,2,4-triazole instead of Cl substitution. Nothing in these references can be construed to disclose or suggest the novel compounds of this invention and their use to inhibit HIV infection.

### REFERENCES CITED

### Patent documents

1. Greenlee, W.J.; Srinivasan, P.C. Indole reverse transcriptase inhibitors. U.S. Patent 5,124,327.
2. Williams, T.M.; Ciccarone, T.M.; Saari, W. S.; Wai, J.S.; Greenlee, W.J.; Balani, S.K.; Goldman, M.E.; Theohrides, A.D. Indoles as inhibitors of HIV reverse transcriptase. European Patent 530907.
3. Romero, D.L.; Thomas, R.C.; Preparation of substituted indoles as anti-AIDS pharmaceuticals. PCT WO 93/01181.
4. Boschelli, D.H.; Connor, D.T.; Unangst, P.C. Indole-2-carboxamides as inhibitors of cell adhesion. U.S. Patent 5,424,329.
5. (a) Mantovanini, M.; Melillo, G.; Daffonchio, L. Tropyl 7-azaindol-3-ylcarboxyamides as antitussive agents. PCT WO 95/04742 (Dompe Spa). (b) Cassidy, F.; Hughes, I.; Rahman, S.; Hunter, D. J. Bisheteroaryl-carbonyl and carboxamide derivatives with 5HT 2C/2B antagonists activity. PCT WO 96/11929. (c) Scherlock, M. H.; Tom, W. C. Substituted 1*H*-pyrrolopyridine-3-carboxamides. U. S. Patent 5,023,265.

### Other Publications

6. Larder, B.A.; Kemp, S.D. Multiple mutations in the HIV-1 reverse transcriptase confer high-level resistance to zidovudine (AZT). Science, 1989, 246,1155-1158.
7. Gulick, R.M. Current antiretroviral therapy: An overview. *Quality of Life Research,* **1997,** *6*, 471-474.
8. Kuritzkes, D.R. HIV resistance to current therapies. Antiviral Therapy, 1997, 2 (Supplement 3), 61-67.
9. Morris-Jones, S.; Moyle, G.; Easterbrook, P.J. Antiretroviral therapies in HIV-1 infection. Expert Opinion on Investigational Drugs, 1997, 6(8),1049-1061.
10. Schinazi, R.F.; Larder, B.A.; Mellors, J.W. Mutations in retroviral genes associated with drug resistance. International Antiviral News, 1997, 5,129-142.
11. Vacca, J.P.; Condra, J.H. Clinically effective HIV-1 protease inhibitors. Drug Discovery Today, 1997, 2, 261-272.
12. Flexner, D. HIV-protease inhibitors. Drug Therapy, 1998, 338, 1281-1292.
13. Berkhout, B. HIV-1 evolution under pressure of protease inhibitors: Climbing the stairs of viral fitness. J. Biomed. Sci., 1999, 6, 298-305.
14. Ren, S.; Lien, E. J. Development of HIV protease inhibitors: A survey. Prog. Drug Res., 1998, 51, 1-31.
15. Pedersen, O.S.; Pedersen, E.B. Non-nucleoside reverse transcriptase inhibitors: the NNRTI boom. Antiviral Chem. Chemother. 1999,10, 285-314.
16. (a) De Clercq, E. The role of non-nucleoside reverse transcriptase inhibitors (NNRTIs) in the therapy of HIV-1 infection. Antiviral Research, 1998, 38, 153-179. (b) De Clercq, E. Perspectives of non-nucleoside reverse transcriptase inhibitors (NNRTIs) in the therapy of HIV infection. IL. Farmaco, 1999, 54, 26-45.
17. Font, M.; Monge, A.; Cuartero, A.; Elorriaga, A.; Martinez-Irujo, J.J.; Alberdi, E.; Santiago, E.; Prieto, I.; Lasarte, J.J.; Sarobe, P. and Borras, F. Indoles and pyrazino[4,5-b]indoles as nonnucleoside analog inhibitors of HIV-1 reverse transcriptase. Eur. J. Med. Chem., 1995, 30, 963-971.
18. Romero, D.L.; Morge, R.A.; Genin, M.J.; Biles, C.; Busso, M,; Resnick, L.; Althaus, I.W.; Reusser, F.; Thomas, R.C and Tarpley, W.G. Bis(heteroaryl)piperazine (BHAP) reverse transcriptase inhibitors: structure-activity relationships of novel substituted indole analogues and the identification of 1-[(5-methanesulfonamido-1H-indol-2-yl)-carbonyl]-4-[3-[1-methylethyl)amino]-pyridinyl]piperazine momomethansulfonate (U-90152S), a second generation clinical candidate. J. Med. Chem., 1993, 36, 1505-1508.
19. Young, S.D.; Amblard, M.C.; Britcher, S.F.; Grey, V.E.; Tran, L.O.; Lumma, W.C.; Huff, J.R.; Schleif, W.A.; Emini, E.E.; O'Brien, J.A.; Pettibone, D.J. 2-Heterocyclic indole-3-sulfones as inhibitors of HIV-reverse transcriptase. Bioorg. Med. Chem. Lett., 1995, 5, 491-496.
20. Genin, M.J.; Poel, T.J.; Yagi, Y.; Biles, C.; Althaus, I.; Keiser, B.J.; Kopta, L.A.; Friis, J.M.; Reusser, F.; Adams, W.J.; Olmsted, R.A.; Voorman, R.L.; Thomas, R.C. and Romero, D.L. Synthesis and bioactivity of novel bis(heteroaryl)piperazine (BHAP) reverse transcriptase inhibitors: structure-activity relationships and increased metabolic stability of novel substituted pyridine analogs. J. Med. Chem., 1996, 39, 5267-5275.
21. Silvestri, R.; Artico, M.; Bruno, B.; Massa, S.; Novellino, E.; Greco, G.; Marongiu, M.E.; Pani, A.; De Montis, A and La Colla, P. Synthesis and biological evaluation of 5H-indolo[3,2-b][1,5]benzothiazepine derivatives, designed as conformationally constrained analogues of the human immunodeficiency virus type 1 reverse transcriptase inhibitor L-737,126. Antiviral Chem. Chemother. 1998, 9, 139-148.
22. Fredenhagen, A.; Petersen, F.; Tintelnot-Blomley, M.; Rosel, J.; Mett, H and Hug, P. J. Semicochliodinol A and B: Inhibitors of HIV-1 protease and EGF-R protein Tyrosine Kinase related to Asterriquinones produced by the fungus Chrysosporium nerdarium. Antibiotics, 1997, 50, 395-401.
23. Kato, M.; Ito, K.; Nishino, S.; Yamakuni, H.; Takasugi, H. New 5-HT3 (Serotonin-3) receptor antagonists. IV. Synthesis and structure-activity relationships of azabicycloalkaneacetamide derivatives. Chem. Pharm. Bull., 1995, 43, 1351-1357.
24. Levacher, V.; Benoit, R.; Duflos, J; Dupas, G.; Bourguignon, J.; Queguiner, G. Broadening the scope of NADH models by using chiral and non chiral pyrrolo [2,3-b] pyridine derivatives. Tetrahedron, 1991, 47, 429-440.
25. Shadrina, L.P.; Dormidontov, Yu.P.; Ponomarev, V,G.; Lapkin, I.I. Reactions of organomagnesium derivatives of 7-aza- and benzoindoles with diethyl oxalate and the reactivity of ethoxalylindoles. Khim. Geterotsikl. Soedin., 1987, 1206-1209.
26. Sycheva, T.V.; Rubtsov, N.M.; Sheinker, Yu.N.; Yakhontov, L.N. Some reactions of 5-cyano-6-chloro-7-azaindoles and lactam-lactim tautomerism in 5-cyano-6-hydroxy-7-azaindolines. Khim. Geterotsikl. Soedin., 1987, 100-106.
27. (a) Desai, M.; Watthey, J.W.H.; Zuckerman, M. A convenient preparation of 1-aroylpiperazines. Org. Prep. Proced. Int., 1976, 8, 85-86. (b) Adamczyk, M.; Fino, J.R. Synthesis of procainamide metabolites. N-acetyl desethylprocainamide and desethylprocainamide. Org. Prep. Proced Int. 1996, 28, 470-474. (c) Rossen, K.; Weissman, S.A.; Sager, J.; Reamer, R.A.; Askin, D.; Volante, R.P.; Reider, P.J. Asymmetric Hydrogenation of tetrahydropyrazines: Synthesis of (S)-piperazine 2-tert-butylcarboxamide, an intermediate in the preparation of the HIV protease inhibitor Indinavir. Tetrahedron Lett., 1995, 36, 6419-6422. (d) Wang, T.; Zhang, Z.; Meanwell, N.A. Benzoylation of Dianions: Preparation of mono-Benzoylated Symmetric Secondary Diamines. J. Org. Chem., 1999, 64, 7661-7662.
28. Li, H.; Jiang, X.; Ye, Y.-H.; Fan, C.; Romoff, T.; Goodman, M. 3-(Diethoxyphosphoryloxy)-1,2,3-benzotriazin-4(3H)-one (DEPBT): A new coupling reagent with remarkable resistance to racemization. Organic Lett., 1999, 1, 91-93.
29. Harada, N.; Kawaguchi, T.; Inoue, I.; Ohashi, M.; Oda, K.; Hashiyama, T.; Tsujihara, K. Synthesis and antitumor activity of quaternary salts of 2-(2'-oxoalkoxy)-9-hydroxyellipticines. Chem. Pharm. Bull., 1997, 45, 134-137.
30. Schneller, S. W.; Luo, J.-K. Synthesis of 4-amino-1H-pyrrolo[2,3-b]pyridine (1,7-Dideazaadenine) and 1H-pyrrolo[2,3-b]pyridin-4-ol (1,7-Dideazahypoxanthine). J. Org. Chem., 1980, 45, 4045-4048.
31. Shiotani, S.; Tanigochi, K. Furopyridines. XXII [1]. Elaboration of the C-substitutents alpha to the heteronitrogen atom of furo[2,3-b]-, -[3.2-b]-, -[2.3-c]- and -[3,2-c]pyridine. J. Het. Chem., 1997, 34, 901-907.
32. Minakata, S.; Komatsu, M.; Ohshiro, Y. Regioselective functionalization of 1H-pyrrolo[2,3-b]pyridine via its N-oxide. Synthesis, 1992, 661-663*.*
33. Klemm, L. H.; Hartling, R. Chemistry of thienopyridines. XXIV. Two transformations of thieno[2,3-b]pyridine 7-oxide (1). J. Het. Chem., 1976, 13, 1197-1200.
34. Antonini, I.; Claudi, F.; Cristalli, G.; Franchetti, P.; Crifantini, M.; Martelli, S. Synthesis of 4-amino-1-□-D-ribofuranosyl-1H-pyrrolo[2,3-b]pyridine (1-Deazatubercidin) as a potential antitumor agent. J. Med. Chem., 1982, 25, 1258-1261.
35. (a) Regnouf De Vains, J.B.; Papet, A.L.; Marsura, A. New symmetric and unsymmetric polyfunctionalized 2,2'-bipyridines. J. Het. Chem., 1994, 31, 1069-1077. (b) Miura, Y.; Yoshida, M.; Hamana, M. Synthesis of 2,3-fused quinolines from 3-substituted quinoline 1-oxides. Part II, Heterocycles, 1993, 36, 1005-1016. (c) Profft, V.E.; Rolle, W. Uber 4-merkaptoverbindungendes 2-methylpyridins. J. Prakt. Chem., 1960, 283 (11), 22-34.
36. Nesi, R.; Giomi, D.; Turchi, S.; Tedeschi, P., Ponticelli, F. A new one step synthetic approach to the isoxazolo[4,5-b]pyridine system. Synth. Comm., 1992, 22, 2349-2355.
37. (a) Walser, A.; Zenchoff, G.; Fryer, R.I. Quinazolines and 1,4-benzodiazepines. 75. 7-Hydroxyaminobenzodiazepines and derivatives. J. Med. Chem., 1976, 19, 1378-1381. (b) Barker, G.; Ellis, G.P. Benzopyrone. Part I. 6-Amino- and 6-hydroxy-2-subtituted chromones. J. Chem. Soc., 1970, 2230-2233.
38. Ayyangar, N.R.; Lahoti, R J.; Daniel, T. An alternate synthesis of 3,4-diaminobenzophenone and mebendazole. Org. Prep. Proced. Int., 1991, 23, 627-631.
39. Mahadevan, I.; Rasmussen, M. Ambident heterocyclic reactivity: The alkylation of pyrrolopyridines (azaindoles, diazaindenes). Tetrahedron, 1993, 49, 7337-7352.
40. Chen, B.K.; Saksela, K.; Andino, R.; Baltimore, D. Distinct modes of human immunodeficiency type 1 proviral latency revealed by superinfection of nonproductively infected cell lines with recombinant luciferase-encoding viruses. J. Virol., 1994, 68, 654-660.
41. Bodanszky, M.; Bodanszky, A. "The Practice of Peptide Synthesis 2nd Ed., Springer-Verlag: Berlin Heidelberg, Germany, 1994.
42. Albericio, F. et al. J. Org. Chem. 1998, 63, 9678.
43. Knorr, R. et al. Tetrahedron Lett. 1989, 30, 1927.
44. (a) Jaszay Z. M. et al. Synth. Commun., 1998 28, 2761 and references cited therein; (b) Bernasconi, S. et al. Synthesis, 1980, 385.
45. (a) Jaszay Z. M. et al. Synthesis, 1989, 745 and references cited therein; (b) Nicolaou, K. C. et al. Angew. Chem. Int. Ed. 1999, 38, 1669.
46. Ooi, T. et al. Synlett. 1999, 729.
47. Ford, R. E. et al. J. Med. Chem. 1986, 29, 538.
48. (a) Yeung, K.-S. et al. Bristol-Myers Squibb Unpublished Results. (b) Wang, W. et al. Tetrahedron Lett. 1999, 40, 2501.
49. Brook, M. A. et al. Synthesis, 1983, 201.
50. Yamazaki, N. et al. Tetrahedron Lett. 1972, 5047.
51. Barry A. Bunin "The Combinatorial Index" 1998 Academic Press, San Diego / London pages 78-82.
52. Richard C. Larock Comprehensive Organic Transormations 2nd Ed. 1999, John Wiley and Sons New York.
53. M.D. Mullican et al. J. Med. Chem. 1991, 34, 2186-2194.
54. Protective groups in organic synthesis 3rd ed. / Theodora W. Greene and Peter G.M. Wuts. New York: Wiley, 1999.
55. Katritzky, Alan R. Lagowski, Jeanne M. The principles of heterocyclic ChemistryNew York: Academic Press, 1968.
56. Paquette, Leo A. Principles of modem heterocyclic chemistry New York : Benjamin.
57. Katritzky, Alan R.; Rees, Charles W.; Comprehensive heterocyclic chemistry: the structure, reactions, synthesis, and uses of heterocyclic compounds 1st ed.Oxford (Oxfordshire) ; New York: Pergamon Press, 1984. 8 v.
58. Katritzky, Alan RHandbook of heterocyclic 1st edOxford (Oxfordshire) ; New York : Pergamon Press, 1985.
59. Davies, David I Aromatic Heterocyclic Oxford ; New York : Oxford University Press, 1991.
60. Ellis, G. P. Synthesis of fused Chichester [Sussex] ; New York: Wiley, c1987-c1992. Chemistry of heterocyclic compounds ; v. 47.
61. Joule, J. A Mills, K. ,Smith, G. F. Heterocyclic Chemistry, 3rd ed London; New York Chapman & Hall, 1995.
62. Katritzky, Alan R., Rees, Charles W. , Scriven, Eric F. V. Comprehensive heterocyclic chemistry II : a review of the literature 1982-1995.
63. The structure, reactions, synthesis, and uses of heterocyclic compounds 1st ed. Oxford ; New York: Pergamon, 1996. 11 v. in 12 : ill. ; 28 cm.
64. Eicher, Theophil, Hauptmann, Siegfried. The chemistry of heterocycles : structure, reactions, syntheses, and applications Stuttgart ; New York : G. Thieme, 1995.
65. Grimmett, M. R. Imidazole and benzimidazole Synthesis London; San Diego: Academic Press, 1997.
66. Advances in heterocyclic chemistry. Published in New York by Academic Press, starting in 1963-present.
67. Gilchrist, T. L. (Thomas Lonsdale) Heterocyclic chemistry 3rd ed. Harlow, Essex : Longman, 1997. 414 p. : ill. ; 24 cm.
68. Farina, Vittorio; Roth, Gregory P. Recent advances in the Stille reaction; Adv. Met.-Org. Chem. 1996, 5, 1-53.
69. Farina, Vittorio; Krishnamurthy, Venkat; Scott, William J. The Stille reaction; Org. React. (N. Y.) (1997), 50, 1-652.
70. Stille, J. K. Angew. Chem. Int. Ed. Engl. 1986, 25, 508-524.
71. Norio Miyaura and Akiro Suzuki Chem Rev. 1995, 95, 2457.
72. Home, D.A. Heterocycles 1994, 39, 139.
73. Kamitori, Y. et al. Heterocycles, 1994, 37(1), 153.
74. Shawali, J. Heterocyclic Chem. 1976, 13, 989.
75. a) Kende, A.S.et al. Org. Photochem. Synth. 1972, 1, 92. b) Hankes, L.V.; Biochem. Prep. 1966, 11, 63. c) Synth. Meth. 22, 837.
76. Hulton et. al. Synth. Comm. 1979, 9, 789.
77. Pattanayak, B.K. et.al. Indian J. Chem. 1978, 16, 1030.
*78.* Chemische Berichte 1902, 35, 1545.
*79.* Chemische Berichte Ibid 1911, 44, 493.
80. Moubarak, I., Vessiere, R. Synthesis 1980, Vol. 1, 52-53.
81. Ind J. Chem. 1973, 11, 1260.
82. Roomi et al. Can J. Chem. 1970, 48,1689.
83. Sorrel, T.N. J. Org. Chem. 1994, 59, 1589.
84. Nitz, T.J. et. al. J. Org. Chem. 1994, 59, 5828-5832.
85. Bowden, K. et.al. J. Chem. Soc. 1946, 953.
86. Nitz, T.J. et. al. J. Org. Chem. 1994, 59, 5828-5832.
87. Scholkopf et. al. Angew. Int. Ed. Engl. 1971, 10(5), 333.
88. (a) Behun, J. D.; Levine, R. J. Org. Chem. 1961, 26, 3379. (b) Rossen, K.; Weissman, S.A.; Sager, J.; Reamer, R.A.; Askin, D.; Volante, R.P.; Reider, P.J. Asymmetric Hydrogenation of tetrahydropyrazines: Synthesis of (S)-piperazine 2-tert-butylcarboxamide, an intermediate in the preparation of the HIV protease inhibitor Indinavir. Tetrahedron Lett., 1995, 36, 6419-6422. (c) Jenneskens, L. W.; Mahy, J.; den Berg, E. M. M. de B.-v.; Van der Hoef, I.; Lugtenburg, J. Recl. Trav. Chim. Pays-Bas 1995, 114, 97.
89. Wang, T.; Zhang, Z.; Meanwell, N.A. Benzoylation of Dianions: Preparation of mono-Benzoylated Symmetric Secondary Diamines. J. Org. Chem., 1999, 64, 7661-7662.
90. (a) Adamczyk, M.; Fino, J.R. Synthesis of procainamide metabolites. N-acetyl desethylprocainamide and desethylprocainamide. Org. Prep. Proced. Int. 1996, 28, 470-474. (b) Wang, T.; Zhang, Z.; Meanwell, N.A. Regioselective mono-Benzoylation of Unsymmetrical Piperazines. J. Org. Chem. 2000, 65, 4740.
91. Masuzawa, K.; Kitagawa, M.; Uchida, H. Bull Chem. Soc. Jpn. 1967, 40, 244-245.
92. Furber, M.; Cooper, M. E.; Donald, D. K. Tetrahedron Lett. 1993, 34, 1351-1354.
93. Blair, Wade S.; Deshpande, Milind; Fang, Haiquan; Lin, Pin-fang; Spicer, Timothy P.; Wallace, Owen B.; Wang, Hui; Wang, Tao; Zhang, Zhongxing; Yeung, Kap-sun. Preparation of antiviral indoleoxoacetyl piperazine derivatives US patent 6,469,006. Preparation of antiviral indoleoxoacetyl piperazine derivatives. PCT Int. Appl. (PCT/US00/14359), WO 0076521 A1, filed May 24, 2000, published December 21, 2000.
94. Wang, Tao; Wallace, Owen B.; Zhang, Zhongxing; Meanwell, Nicholas A.; Bender, John A. Antiviral azaindole derivatives. U.S. patent 6476034 and Wang, Tao; Wallace, Owen B.; Zhang, Zhongxing; Meanwell, Nicholas A.; Bender, John A. Preparation of antiviral azaindole derivatives. PCT Int. Appl. (PCT/US01/02009), WO 0162255 A1, filed January 19, 2001, published August 30, 2001.
95. Wallace, Owen B.; Wang, Tao; Yeung, Kap-Sun; Pearce, Bradley C.; Meanwell, Nicholas A.; Qiu, Zhilei; Fang, Haiquan; Xue, Qiufen May; Yin, Zhiwei. Composition and antiviral activity of substituted indoleoxoacetic piperazine derivatives. U.S. Patent Application Serial Number 10/027,612 filed December 19, 2001, which is a continuation-in-part application of U.S. Serial Number 09/888,686 filed June 25, 2001 (corresponding to PCT Int. Appl. (PCT/US01/20300), WO 0204440 A1, filed June 26, 2001, published January 17, 2002.
96. J. L. Marco, S. T. Ingate, and P. M. Chinchon Tetrahedron 1999, 55, 7625-7644.
97. C. Thomas, F. Orecher, and P.Gmeiner Synthesis 1998, 1491.
98. M. P. Pavia, S. J. Lobbestael, C. P. Taylor, F. M. Hershenson, and D. W. Miskell
99. Buckheit, Robert W., Jr. Expert Opinion on Investigational Drugs 2001, 10(8), 1423-1442.
100. Balzarini, J.; De Clercq, E.. Antiretroviral Therapy 2001, 31-62.
101. E. De clercq Journal of Clinical Virology, 2001, 22,73-89.
102. Merour, Jean-Yves; Joseph, Benoit. Curr. Org. Chem. (2001), 5(5), 471-506.
103. T. W. von Geldern et al. J. Med. Chem 1996, 39, 968.
104. M. Abdaoui et al. Tetrahedron 2000, 56, 2427.
105. W. J. Spillane et al. J. Chem. Soc., Perkin Trans. 1, 1982, 3, 677
106. Wang, Tao; Wallace, Owen B.; Zhang, Zhongxing; Meanwell, Nicholas A.; Kadow, John F. Yin, Zhiwei. Composition and Antiviral Activity of Substituted Azaindoleoxoacetic Piperazine Derivatives. U.S. Patent Application Serial Number 10/214,982 filed August 7, 2002, which is a continuation-in-part application of U.S. Serial Number 10/038,306 filed January 2, 2002 (corresponding to PCT Int. Appl. (PCT/US02/00455), WO 02/062423 A1, filed January 2, 2002, published August 15, 2002.

### SUMMARY OF THE INVENTION

The invention relates to the compound, including pharmaceutically acceptable salts thereof, 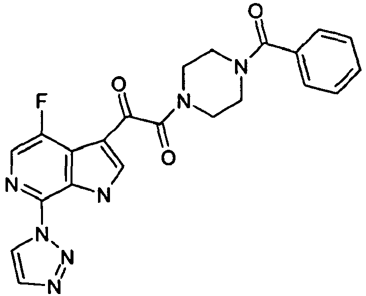 or to the compound, including pharmaceutically acceptable salts thereof,

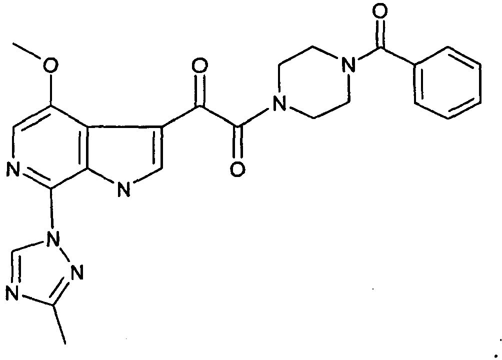

An embodiment of the invention is a pharmaceutical formulation comprising an antiviral effective amount of a compound of the invention, including pharmaceutically acceptable salts thereof and a pharmaceutically acceptable carrier. When used for treating HIV infection, said formulation can optionally additionally contain an antiviral effective amount of an AIDS treatment agent selected from the group consisting of: an AIDS antiviral agent; an antiinfective agent; an immunomodulator; and HIV entry inhibitors.

A further embodiment of the invention is compound of the invention for use in a method for treating mammals infected with a virus, such as HIV, comprising administering to said mammal an antiviral effective amount of a compound of the invention, including pharmaceutically acceptable salts thereof, a pharmaceutically acceptable carrier, optionally in combination with an antiviral effective amount of an AIDS treatment agent selected from the group consisting of: (a) an AIDS antiviral agent; (b) an anti-infective agent; (c) an immunomodulator; and (d) HIV entry inhibitors.

### DETAILED DESCRIPTION OF THE INVENTION

Since the compounds of the present invention, may possess asymmetric centers and therefore occur as mixtures of diastereomers and enantiomers, the present disclosure includes the individual diastereoisomeric and enantiomeric forms of the compounds of the invention in addition to the mixtures thereof.

Physiologically acceptable salts of compounds disclosed herein are within the scope of this invention. The term "pharmaceutically acceptable salt" as used herein and in the claims is intended to include nontoxic base addition salts. Suitable salts include those derived from organic and inorganic acids such as, without limitation, hydrochloric acid, hydrobromic acid, phosphoric acid, sulfuric acid, methanesulfonic acid, acetic acid, tartaric acid, lactic acid, sulfinic acid, citric acid, maleic acid, fumaric acid, sorbic acid, aconitic acid, salicylic acid, phthalic acid, and the like. The term "pharmaceutically acceptable salt" as used herein is also intended to include salts of acidic groups, such as a carboxylate, with such counterions as ammonium, alkali metal salts, particularly sodium or potassium, alkaline earth metal salts, particularly calcium or magnesium, and salts with suitable organic bases such as lower alkylamines (methylamine, ethylamine, cyclohexylamine, and the like) or with substituted lower alkylamines (e.g. hydroxyl-substituted alkylamines such as diethanolamine, triethanolamine or tris(hydroxymethyl)- aminomethane), or with bases such as piperidine or morpholine.

In the present invention, the term "antiviral effective amount" means the total amount of each active component that is sufficient to show a meaningful patient benefit, i.e., healing of acute conditions characterized by inhibition of the HIV infection. When applied to an individual active ingredient, administered alone, the term refers to that ingredient alone. When applied to a combination, the term refers to combined amounts of the active ingredients that result in the therapeutic effect, whether administered in combination, serially or simultaneously. The terms "treat, treating, treatment" as used herein and in the claims means preventing or ameliorating diseases associated with HIV infection.

The present invention is also directed to combinations of the compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those in the following table.

### ANTIVIRALS

| Drug Name | Manufacturer | Indication |
|---|---|---|
| 097 | Hoechs/Bayer | HIV infection, AIDS, ARC (non-nucleoside reverse transcriptase (RT) inhibitor) |
| | | |
| Amprenivir | Glaxo Wellcome | HIV infection, |
| 141 W94 | | AIDS, ARC |
| GW 141 | | (protease inhibitor) |
| | | |
| Abacavir (1592U89) | Glaxo Wellcome | HIV infection, |
| GW 1592 | | AIDS, ARC (RT inhibitor) |
| | | |
| Acemannan | Carrington Labs (Irving, TX) | ARC |
| Acyclovir | Burroughs Wellcome | HIV infection, AIDS, ARC, in combination with AZT |
| | | |
| AD-439 | Tanox Biosystems | HIV infection, AIDS, ARC |
| | | |
| AD-519 | Tanox Biosystems | HIV infection, AIDS, ARC |
| | | |
| Adefovir dipivoxil | Gilead Sciences | HIV infection |
| AL-721 | Ethigen | ARC, PGL |
| | (Los Angeles, CA) | HIV positive, AIDS |
| | | |
| Alpha Interferon | Glaxo Wellcome | Kaposi's sarcoma, HIV in combination w/Retrovir |
| Ansamycin | Adria Laboratories | ARC |
| LM 427 | (Dublin, OH) Erbamont (Stamford, CT) | |
| | | |
| Antibody which | Advanced Biotherapy | AIDS, ARC |
| Neutralizes pH | Concepts | |
| Labile alpha aberrant | (Rockville, MD) | |
| Interferon | | |
| | | |
| AR177 | Aronex Pharm | HIV infection, AIDS, ARC |
| | | |
| Beta-fluoro-ddA | Nat'l Cancer Institute | AIDS-associated diseases |
| | | |
| BMS-232623 | Bristol-Myers Squibb/ | HIV infection, |
| (CGP-73547) | Novartis | AIDS, ARC (protease inhibitor) |
| | | |
| BMS-234475 | Bristol-Myers Squibb/ | HIV infection, |
| (CGP-61755) | Novartis | AIDS, ARC (protease inhibitor) |
| | | |
| CI-1012 | Warner-Lambert | HIV-1 infection |
| | | |
| Cidofovir | Gilead Science | CMV retinitis, herpes, papillomavirus |
| | | |
| Curdlan sulfate | AJI Pharma USA | HIV infection |
| | | |
| Cytomegalovirus | MedImmune | CMV retinitis |
| Immune globin | | |
| | | |
| Cytovene | Syntex | Sight threatening |
| Ganciclovir | | CMV |
| | | peripheral CMV |
| | | retinitis |
| Delaviridine | Pharmacia-Upjohn | HIV infection, AIDS, ARC (RT inhibitor) |
| | | |
| Dextran Sulfate | Ueno Fine Chem. Ind. Ltd. (Osaka, Japan) | AIDS, ARC, HIV positive asymptomatic |
| | | |
| ddC | Hoffman-La Roche | HIV infection, AIDS, |
| Dideoxycytidine | | ARC |
| | | |
| ddI Dideoxyinosine | Bristol-Myers Squibb | HIV infection, AIDS, ARC; combination with AZT/d4T |
| | | |
| DMP-450 | AVID (Camden, NJ) | HIV infection, AIDS, ARC (protease inhibitor) |
| | | |
| Efavirenz (DMP 266) (-)6-Chloro-4-(S)-cyclopropylethynyl-4(S)-trifluoromethyl-1,4-dihydro-2H-3,1-benzoxazin-2-one, STOCRINE | DuPont Merck | HIV infection, AIDS, ARC (non-nucleoside RT inhibitor) |
| | | |
| EL10 | Elan Corp, PLC (Gainesville, GA) | HIV infection |
| | | |
| Famciclovir | Smith Kline | herpes zoster, herpes simplex |
| | | |
| FTC | Emory University | HIV infection, AIDS, ARC (reverse transcriptase inhibitor) |
| GS 840 | Gilead | HIV infection, AIDS, ARC (reverse transcriptase inhibitor) |
| | | |
| HBY097 | Hoechst Marion Roussel | HIV infection, AIDS, ARC (non-nucleoside reverse transcriptase inhibitor) |
| | | |
| Hypericin | VIMRx Pharm. | HIV infection, AIDS, ARC |
| | | |
| Recombinant Human Interferon Beta | Triton Biosciences (Almeda, CA) | AIDS, Kaposi's sarcoma, ARC |
| | | |
| Interferon alfa-n3 | Interferon Sciences | ARC, AIDS |
| | | |
| Indinavir | Merck | HIV infection, AIDS, ARC, asymptomatic HIV positive, also in combination with AZT/ddT/ddC |
| | | |
| ISIS 2922 | ISIS Pharmaceuticals | CMV retinitis |
| | | |
| KNI-272 | Nat'l Cancer Institute | HIV-assoc. diseases |
| | | |
| Lamivudine, 3TC | Glaxo Wellcome | HIV infection, AIDS, ARC (reverse transcriptase inhibitor); also with AZT |
| Lobucavir | Bristol-Myers Squibb | CMV infection |
| Nelfinavir | Agouron | HIV infection, |
| | Pharmaceuticals | AIDS, ARC (protease inhibitor) |
| | | |
| Nevirapine | Boeheringer | HIV infection, |
| | Ingleheim | AIDS, ARC (RT inhibitor) |
| | | |
| Novapren | Novaferon Labs, Inc. (Akron, OH) | HIV inhibitor |
| | | |
| Peptide T | Peninsula Labs | AIDS |
| Octapeptide | (Belmont, CA) | |
| Sequence | | |
| | | |
| Trisodium | Astra Pharm. | CMV retinitis, HIV |
| Phosphonoformate | Products, Inc. | infection, other CMV |
| | | infections |
| | | |
| PNU-140690 | Pharmacia Upjohn | HIV infection, |
| | | AIDS, ARC (protease inhibitor) |
| | | |
| Probucol | Vyrex | HIV infection, AIDS |
| | | |
| RBC-CD4 | Sheffield Med. | HIV infection, |
| | Tech (Houston, TX) | AIDS, ARC |
| | | |
| Ritonavir | Abbott | HIV infection, |
| | | AIDS, ARC (protease inhibitor) |
| | | |
| Saquinavir | Hoffmann-LaRoche | HIV infection, AIDS, ARC (protease inhibitor) |
| Stavudine; d4T Didehydrodeoxy-thymidine | Bristol-Myers Squibb | HIV infection, AIDS, ARC |
| | | |
| Valaciclovir | Glaxo Wellcome | Genital HSV & CMV infections |
| | | |
| Virazole | Viratek/ICN | asymptomatic HIV |
| Ribavirin | (Costa Mesa, CA) | positive, LAS, ARC |
| | | |
| VX-478 | Vertex | HIV infection, AIDS, ARC |
| | | |
| Zalcitabine | Hoffmann-LaRoche | HIV infection, AIDS, ARC, with AZT |
| | | |
| Zidovudine; AZT | Glaxo Wellcome | HIV infection, AIDS, ARC, Kaposi's sarcoma, in combination with other therapies |
| | | |
| Tenofovir disoproxil, | Gilead | HIV infection, |
| fumarate salt (Viread^{®}) | | AIDS, (reverse transcriptase inhibitor) |
| | | |
| Combivir^{®} | GSK | HIV infection, |
| | | AIDS, (reverse transcriptase inhibitor) |
| | | |
| abacavir succinate | GSK | HIV infection, |
| (or Ziagen^{®}) | | AIDS, (reverse transcriptase inhibitor) |
| | | |
| REYATAZ^{®} | Bristol-Myers Squibb | HIV infection |
| (or atazanavir) | | AIDs, protease inhibitor |
| | | |
| FUZEON | Roche / Trimeris | HIV infection |
| (or T-20) | | AIDS, viral Fusion inhibitor |

### IMMUNOMODULATORS

| Drug Name | Manufacturer | Indication |
|---|---|---|
| AS-101 | Wyeth-Ayerst | AIDS |
| | | |
| Bropirimine | Pharmacia Upjohn | Advanced AIDS |
| | | |
| Acemannan | Carrington Labs, Inc. (Irving, TX) | AIDS, ARC |
| | | |
| CL246,738 | American Cyanamid Lederle Labs | AIDS, Kaposi's sarcoma |
| | | |
| EL10 | Elan Corp, PLC (Gainesville, GA) | HIV infection |
| | | |
| FP-21399 | Fuki ImmunoPharm | Blocks HIV fusion with CD4+ cells |
| | | |
| Gamma Interferon | Genentech | ARC, in combination w/TNF (tumor necrosis factor) |
| | | |
| Granulocyte Macrophage Colony Stimulating Factor | Genetics Institute Sandoz | AIDS |
| | | |
| Granulocyte Macrophage Colony Stimulating Factor | Hoechst-Roussel Immunex | AIDS |
| | | |
| Granulocyte Macrophage Colony Stimulating Factor | Schering-Plough | AIDS, combination w/AZT |
| HIV Core Particle Immunostimulant | Rorer | Seropositive HIV |
| | | |
| IL-2 Interleukin-2 | Cetus | AIDS, in combination w/AZT |
| | | |
| IL-2 Interleukin-2 | Hoffman-LaRoche Immunex | AIDS, ARC, HIV, in combination w/AZT |
| | | |
| IL-2 Interleukin-2 (aldeslukin) | Chiron | AIDS, increase in CD4 cell counts |
| | | |
| Immune Globulin Intravenous (human) | Cutter Biological (Berkeley, CA) | Pediatric AIDS, in combination w/AZT |
| | | |
| IMREG-1 | Imreg (New Orleans, LA) | AIDS, Kaposi's sarcoma, ARC, PGL |
| | | |
| IMREG-2 | Imreg (New Orleans, LA) | AIDS, Kaposi's sarcoma, ARC, PGL |
| | | |
| Imuthiol Diethyl Dithio Carbamate | Merieux Institute | AIDS, ARC |
| | | |
| Alpha-2 Interferon | Schering Plough | Kaposi's sarcoma w/AZT, AIDS |
| | | |
| Methionine-Enkephalin | TNI Pharmaceutical (Chicago, IL) | AIDS, ARC |
| | | |
| MTP-PE | Ciba-Geigy Corp. | Kaposi's sarcoma |
| | | |
| Muramyl-Tripeptide | Amgen | AIDS, in combination |
| Granulocyte | | w/AZT |
| Colony Stimulating | | |
| Factor | | |
| | | |
| Remune | Immune Response Corp. | Immunotherapeutic |
| rCD4 | Genentech | AIDS, ARC |
| Recombinant | | |
| Soluble Human CD4 | | |
| | | |
| rCD4-IgG | | AIDS, ARC |
| hybrids | | |
| | | |
| Recombinant | Biogen | AIDS, ARC |
| Soluble Human CD4 | | |
| | | |
| Interferon | Hoffman-La Roche | Kaposi's sarcoma |
| Alfa 2a | | AIDS, ARC, in combination w/AZT |
| | | |
| SK&F106528 | Smith Kline | HIV infection |
| Soluble T4 | | |
| | | |
| Thymopentin | Immunobiology | HIV infection |
| | Research Institute | |
| | (Annandale, NJ) | |
| | | |
| Tumor Necrosis | Genentech | ARC, in combination |
| Factor; TNF | | w/gamma Interferon |
| | | |

| ANTI-INFECTIVES | | |
|---|---|---|
| Drug Name | Manufacturer | Indication |
| Clindamycin with | Pharmacia Upjohn | PCP |
| Primaquine | | |
| | | |
| Fluconazole | Pfizer | Cryptococcal |
| | | meningitis, |
| | | candidiasis |
| | | |
| Pastille | Squibb Corp. | Prevention of |
| Nystatin Pastille | | oral candidiasis |
| | | |
| Ornidyl Eflornithine | Merrell Dow | PCP |
| Pentamidine | LyphoMed | PCP treatment |
| Isethionate (IM & IV) | (Rosemont, IL) | |
| | | |
| Trimethoprim | | Antibacterial |
| | | |
| Trimethoprim/sulfa | | Antibacterial |
| | | |
| Piritrexim | Burroughs Wellcome | PCP treatment |
| | | |
| Pentamidine | Fisons Corporation | PCP prophylaxis |
| Isethionate for | | |
| Inhalation | | |
| | | |
| Spiramycin | Rhone-Poulenc | Cryptosporidial |
| | diarrhea | |
| | | |
| Intraconazole- | Janssen-Pharm. | Histoplasmosis; |
| R51211 | | cryptococcal |
| | | meningitis |
| | | |
| Trimetrexate | Warner-Lambert | PCP |
| | | |
| Daunorubicin | NeXstar, Sequus | Kaposi's sarcoma |
| | | |
| Recombinant Human | Ortho Pharm. Corp. | Severe anemia |
| Erythropoietin | | assoc. with AZT therapy |
| | | |
| Recombinant Human Growth Hormone | Serono | AIDS-related wasting, cachexia |
| | | |
| Megestrol Acetate | Bristol-Myers Squibb | Treatment of anorexia assoc. W/AIDS |
| | | |
| Testosterone | Alza, Smith Kline | AIDS-related wasting |
| Total Enteral Nutrition | Norwich Eaton Pharmaceuticals | Diarrhea and malabsorption related to AIDS |

Additionally, the compounds of the invention herein may be used in combination with another class of agents for treating AIDS which are called HIV entry inhibitors. Examples of such HIV entry inhibitors are discussed in DRUGS OF THE FUTURE 1999, 24(12), pp. 1355-1362; CELL, Vol. 9, pp. 243-246, Oct. 29, 1999; and DRUG DISCOVERY TODAY, Vol. 5, No. 5, May 2000, pp. 183-194.

It will be understood that the scope of combinations of the compounds of this invention with AIDS antivirals, immunomodulators, anti-infectives, HIV entry inhibitors or vaccines is not limited to the list in the above Table, but includes in principle any combination with any pharmaceutical composition useful for the treatment of AIDS.

Preferred combinations are simultaneous or alternating treatments of with a compound of the present invention and an inhibitor of HIV protease and/or a non-nucleoside inhibitor of HIV reverse transcriptase. An optional fourth component in the combination is a nucleoside inhibitor of HIV reverse transcriptase, such as AZT, 3TC, ddC or ddI. A preferred inhibitor of HIV protease is indinavir, which is the sulfate salt of N-(2(R)-hydroxy-1-(S)-indanyl)-2(R)-phenylmethyl-4-(S)-hydroxy-5-(1-(4-(3-pyridyl-methyl)-2(S)-N-(t-butylcarboxamido)-piperazinyl))-pentaneamide ethanolate, and is synthesized according to U.S. 5,413,999. Indinavir is generally administered at a dosage of 800 mg three times a day. Other preferred protease inhibitors are nelfinavir and ritonavir. Another preferred inhibitor of HIV protease is saquinavir which is administered in a dosage of 600 or 1200 mg tid. Preferred non-nucleoside inhibitors of HIV reverse transcriptase include efavirenz. The preparation of ddC, ddI and AZT are also described in EPO 0,484,071. These combinations may have unexpected effects on limiting the spread and degree of infection of HIV. Preferred combinations include those with the following (1) indinavir with efavirenz, and, optionally, AZT and/or 3TC and/or ddI and/or ddC; (2) indinavir, and any of AZT and/or ddI and/or ddC and/or 3TC, in particular, indinavir and AZT and 3TC; (3) stavudine and 3TC and/or zidovudine; (4) zidovudine and lamivudine and 141W94 and 1592U89; (5) zidovudine and lamivudine.

In such combinations the compound of the present invention and other active agents may be administered separately or in conjunction. In addition, the administration of one element may be prior to, concurrent to, or subsequent to the administration of other agent(s).

The preparative procedures and anti-HIV-1 activity of compounds of the invention are summarized bellow.

### Abbreviations

The following abbreviations, most of which are conventional abbreviations well known to those skilled in the art, are used throughout the description of the invention and the examples. Some of the abbreviations used are as follows:
- h: = hour(s)
- rt: = room temperature
- mol: = mole(s)
- mmol: = millimole(s)
- g: = gram(s)
- mg: = milligram(s)
- mL: = milliliter(s)
- TFA: = Trifluoroacetic Acid
- DCE: = 1,2-Dichloroethane
- CH₂Cl₂: = Dichloromethane
- TPAP: = tetrapropylammonium perruthenate
- THF: = Tetrahydofuran
- DEPBT: = 3-(Diethoxyphosphoryloxy)-1,2,3-benzodiazin-4(3H)-one
- DMAP: = 4-dimethylaminopyridine
- P-EDC: = Polymer supported 1-(3-dimethylaminopropyl)-3-ethylcarbodiimide
- EDC: = 1-(3-dimethylaminopropyl)-3-ethylcarbodiimide
- DMF: = *N*,*N*-dimethylformamide
- Hunig's Base: = *N,N*-Diisopropylethylamine
- mCPBA: = *meta*-Chloroperbenzoic Acid
- azaindole: = 1*H*-Pyrrolo-pyridine
- 4-azaindole: = 1*H*-pyrrolo[3,2-b]pyridine
- 5-azaindole: = 1*H*-Pyrrolo[3,2-c]pyridine
- 6-azaindole: = 1*H*-pyrrolo[2,3-c]pyridine
- 7-azaindole: = 1*H*-Pyrrolo[2,3-b]pyridine
- PMB: = 4-Methoxybenzyl
- DDQ: = 2, 3-Dichloro-5, 6-dicyano-1, 4-benzoquinone
- OTf: = Trifluoromethanesulfonoxy
- NMM: = 4-Methylmorpholine
- PIP-COPh: = 1-Benzoylpiperazine
- NaHMDS: = Sodium hexamethyldisilazide
- EDAC: = 1-(3-Dimethylaminopropyl)-3-ethylcarbodiimide
- TMS: = Trimethylsilyl
- DCM: = Dichloromethane
- DCE: = Dichloroethane
- MeOH: = Methanol
- THF: = Tetrahrdrofuran
- EtOAc: = Ethyl Acetate
- LDA: = Lithium diisopropylamide
- TMP-Li: = 2,2,6,6-tetramethylpiperidinyl lithium
- DME: = Dimethoxyethane
- DIBALH: = Diisobutylaluminum hydride
- HOBT: = 1-hydroxybenzotriazole
- CBZ: = Benzyloxycarbonyl
- PCC: = Pyridinium chlorochromate
- Me: = Methyl
- Ph: = Phenyl

### Chemistry

The present invention comprises compounds of the invention, their pharmaceutical formulations, and their use in patients suffering from or susceptible to HIV infection. The compounds of the invention include pharmaceutically acceptable salts thereof.

General procedures to construct substituted azaindole piperazine diamides of the invention and intermediates useful for their synthesis are described in the following Scheme.

Scheme 1 is a preferred method for the preparation of compound Example 1. The synthesis of 2-hydroxy-3-nitro-5-fluoropyridine **5-80** as shown was carried out generally via the methods of A. Marfat and R.P. Robinson U.S. Patent 5,811,432 (column 25, example 5) and Nesnow and Heidleberger (J. Heterocyclic Chem. 1973, 10, pg 779) except that a number of procedural enhancements were incorporated as noted in the description of each step. 2-Hydroxy 5-fluoropyridine **4-80** is also commercially available. The formation of diazonium tetrafluoroborate salt **2-80** from 5-amino-2-methoxy pyridine **1-80** proceeded in essentially quantitative yield and was isolated via filtration. The Schiemann reaction provided poor yields of the desired 2-methoxy-fluoropyridine using the literature conditions due mainly to significant contamination with 3-fluoro 1-(N)-methyl pyridone and other byproducts. However, adoption of a procedure similar to that described in Sanchez, J. P.; Rogowski, J. W.; J Heterocycl Chem 1987, 24, 215 for a related compound provided very high yields of essentially clean but volatile 2-methoxy-5-fluoro pyridine **3-80** as a solution in toluene. In the interest of expediency, demethylation was achieved on large scale using aqueous HCl in pressure bottles at 140°C for 1hr. Prior to heating, the toluene solution was stirred with the aq HCl and then the toluene was removed by decantation. The literature method for carrying out this step using HBr at 100°C was also successful on small scale and had the advantage of avoiding the use of pressure bottles. Nitration of **4-80** as described by Marfat provided lower than expected yields so the procedure was modified slightly, using guidance from A.G. Burton, P.J.Hallis, and A.R. Katritzky (Tetrahedron Letters 1971, 24, 2211-2212) on the control of the regiochemistry of nitration of pyridones via modulation of the acidity of the medium. The chemical yields of 2-hydroxy-3-nitro-5-fluoro pyridine **5-80** were significantly improved using the procedure described in the experimental section. Occasionally the product failed to precipitate during workup and then considerable efforts were necessary to isolate this highly water soluble compound from the aqueous layer. Using neat excess POBr₃, compound **5-80** was converted to 2-bromo-3-nitro-5-fluoro pyridine 6 which could be used without further purification in the subsequent azaindole forming reaction. Addition of the pyridine 6 to excess vinyl magnesium bromide in THF at low temperature afforded the desired 4-fluoro-7-bromo-6-azaindol (precursor 2i) in yields of up to 35% following acidic work up and isolation via crystallization. A disadvantage of this method is the workup is difficult due to the large amounts of salts formed as co-products in the reaction and the low conversion to albeit clean product. The reaction is also exothermic and thus would require care on larger scales. Despite the moderate yields, as mentioned above the reaction proceeds cleanly and provides pure product precursor 2i without chromatography so it is anticipated that more detailed studies of this chemistry could result in yield enhancements. A selective copper/potassium carbonate mediated displacement of the 7-bromo group by commercially available 1,2,3-triazole provided an approximately 1:1 mixture of triazoles from which the desired **7-80** was isolated via chromatography in 25-35% yields. Copper-bronze rather than copper powder can also be used to carry out similar transformations. This reaction must not be allowed to overheat since concomitant displacement of the fluorine is possible and has been observed. Acylation occurred most efficiently under conditions that utilized excess acidic imidazolium chloro aluminate ionic liquid to provide highly activated glyoxylating reagent (K.S. Yeung et al. Tetrahedron Lett. 2002, 43, 5793). The acylation of **7-80** usually does not proceed to completion and typically results in about 75% conversion as measured by LC/MS. An advantage to these conditions is that the typical next step, ester hydrolysis, proceeded *in situ* to provide the desired acid **8-80** which was isolated directly by precipitation during workup. Coupling of the piperazine benzamide was found to be cleaner and produced higher yields of the compound of **Example 1** using the depicted HATU based coupling than with other standard coupling reagents such as EDC or DEPBT.

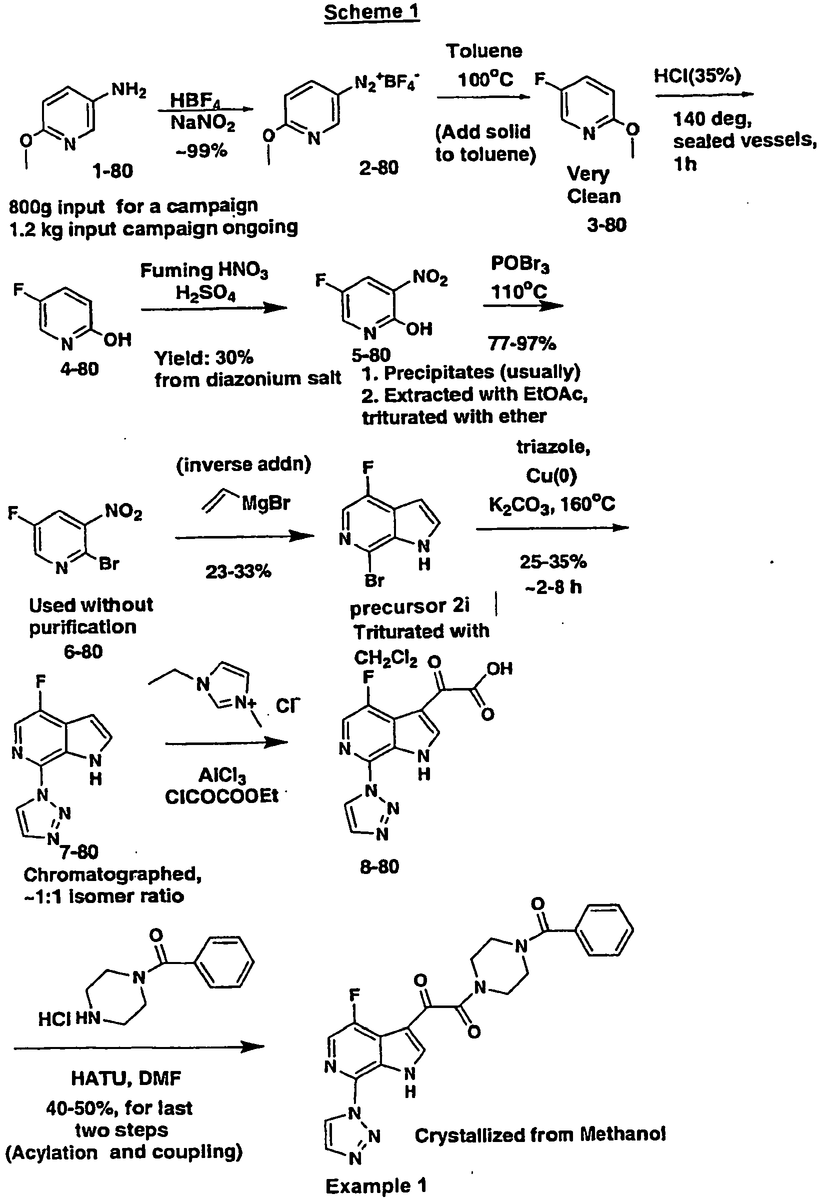

### Precursor 2a

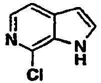

*Typical procedure for preparing azaindole from nitropyridine:* Preparation of 7-chloro-6-azaindole, Precursor 2a, 2-chloro-3-nitropyridine (5.0g, 31.5mmol) was dissolved in dry THF (200 mL). After the solution was cooled to -78 °C, vinyl magnesium bromide (1.0M in THF, 100 mL) was added dropwise. The reaction temperature was maintained at-78°C for 1 h, and then at -20 °C for another 12 h before it was quenched by addition of 20% NH₄Cl aqueous solution (150 mL). The aqueous phase was extracted with EtOAc (3 x 150 mL). The combined organic layer was dried over MgSO₄ , filtered and the filtrate was concentrated *in vacuo* to give a residue which was purified by silica gel column chromatography (EtOAc / Hexane, 1 / 10) to afford 1.5g (31%) of 7-chloro-6-azaindole, Precursor 2a. ¹H NMR (500 MHz, CD₃OD) δ 7.84 (d, 1H, *J* = 10.7 Hz), 7.55 (dd, 1H, *J* = 10.9, 5.45 Hz), 6.62 (d, 1H, *J* = 5.54 Hz), 4.89 (s, 1H). MS *m*/z: (M+H)⁺ calcd for C₇H₆ClN₂: 153.02; found 152.93. HPLC retention time: 0.43 minutes (column A).

### Precursor 2d

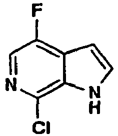

Precursor 2d, 4-fluoro-7-chloro-6-azaindole (above), was prepared according to the following scheme: 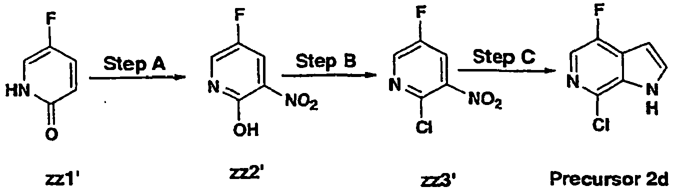
A) fuming HNO₃, H₂SO₄;
B) POCl₃/DMF, 110°C;
C) vinylmagnesium bromide, THF, -78°C ∼ -20°C

It should be noted that 2-chloro-5-fluoro-3-nitro pyridine, zz3', may be prepared by the method in example 5B of the reference Marfat, A. ; and Robinson, R. P. ; "Azaoxindole Derivatives" US. Patent 5,811,432 1998. The preparation below provides some details which enhance the yields of this route.

In Step A, compound zz1' (1.2 g, 0.01 mol) was dissolved in sulfuric acid (2.7 mL) at room temperature. Premixed fuming nitric acid (1 mL) and sulfuric acid was added dropwise at 5-10 °C to the solution of compound zz1'. The reaction mixture was then heated at 85 °C for 1 hour, then was cooled to room temperature and poured into ice (20 g). The yellow solid precipitate was collected by filtration, washed with water and air dried to provide 1.01 g of compound zz2'.

In Step B, compound zz2' (500 mg, 3.16 mmol) was dissolved in phosphorous oxychloride (1.7 mL, 18.9 mmol) and dimethoxyethane at room temperature. The reaction was heated to 110 °C for 5 hours. The excess phosphorous oxychloride was then removed by concentrating the reaction mixture in vacuo. The residue was chromatographed on silica gel, eluted with chloroform (100%) to afford 176 mg of product zz3'.

In Step C, compound zz3' (140 mg, 0.79 mmol) was dissolved in THF (5 mL) and cooled to -78 °C under a nitrogen atmosphere. To this solution was added dropwise a solution of vinyl magnesium bromide (1.2 mmol, 1.0 M in diethyl ether, 1.2 mL). The reaction mixture was then kept at -20 °C for 15 hours. The reaction mixture was then quenched with saturated ammonium chloride, and extracted with ethyl acetate. The combined organic layers were washed with brine, dried over magnesium sulfate, filtered, and the filtrate was concentrated in vacuo. The residue was chromatographed on silica to provide 130 mg of precursor 2d ¹H NMR (500 MHz, CD₃OD) δ 7.78 (s, 1H), 7.60 (d, 1H, *J* = 3.0 Hz), 6.71 (d, 1H, *J* = 3.05 Hz). MS *m*/z: (M+H)⁺ calcd for C₇H₅ClFN₂: 171.10; found 171.00. HPLC retention time: 1.22 minutes (column A).

Precursor 2d, 4-fluoro-7-chloro-6-azaindole, was prepared by the same method as Precursor 2a, starting from 2-Chloro-3-nitro-5-fluoro-pyridine which was prepared according to the procedure above. Experimental details for this preparation are contained in Wang et. al. PCT WO 01/62255. ¹H NMR (500 MHz, CD₃OD δ 7.78 (s, 1H), 7.60 (d, 1H, *J* = 3.0 Hz), 6.71 (d, *1H, J* = 3.05 Hz). MS *m*/z: (M+H)⁺ calcd for C₇H₅ClFN₂: 171.10; found 171.00. HPLC retention time: 1.22 minutes (column A).

### Precursor 2e

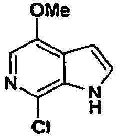

Precursor 2e was prepared by either Method A or Method B, below:

Method A: A mixture of 4-bromo-7-chloro-6-azaindole (1 g), CuI (0.65 g) and NaOMe (4 mL, 25% in methanol) in MeOH (16 mL) was heated at 110 - 120 °C for 16 hours in a sealed tube. After cooling to room temperature, the reaction mixture was neutralized with 1N HCl to pH 7. The aqueous solution was extracted with EtOAc (3 x 30 mL). Then the combined organic layer was dried over MgSO₄, filtered and the filtrate was concentrated *in vacuo* to afford a residue, which was purified by using silica gel chromotography to give 0.3 g of 4-methoxy-7-chloro-6-azaindole, Precursor 2e. MS *m*/z: (M+H)⁺ calcd for C₈H₈ClN₂O: 183.03; found 183.09. HPLC retention time: 1.02 minutes (column B).

Method B :A mixture of 4-bromo-7-chloro-6-azaindole (6 g), CuBr (3.7 g) and NaOMe (30 mL, 5% in MeOH) was heated at 110°C for 24 hours in a sealed tube. After cooling to room temperature, the reaction mixture was added to saturated aqueous NH₄Cl. The resulting aqueous solution was extracted with EtOAc (3 x 30 mL). The combined organic layer was dried over MgSO₄, filtered and the filtrate was concentrated *in vacuo* to afford a residue, which was purified by using silica gel chromotography to give 1.8 g of 4-methoxy-7-chloro-6-azaindole, Precursor 2e.

### Precursor 2i

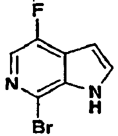

Precursor 2i, 4-fluoro-7-bromo-6-azaindole, was prepared by the same method as Precursor 2e, using POBr₃ in the step B instead of POCl₃. MS *m*/*z:* (M+H)⁺ calcd for C₇H₅BrFN₂: 214.96; found 214.97. HPLC retention time: 1.28 minutes (column G).

### Precursor 3a

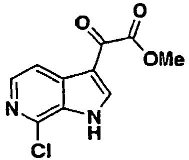

*Typical procedure for acylation of azaindole:* Preparation of Methyl (7-chloro-6-azaindol-3-yl)-oxoacetate. 7-Chloro-6-azaindole, Precursor 2a (0.5 g, 3.3 mmol) was added to a suspension of AlCl₃ (2.2 g, 16.3 mmol) in CH₂Cl₂ (100 mL). Stirring was continued at rt for 10 minutes before methyl chlorooxoacetate (2.0 g, 16.3 mmol) was added dropwise. The reaction was stirred for 8 h. The reaction was quenched with iced aqueous NH₄OAc solution (10%, 200 mL). The aqueous phase was extracted with CH₂Cl₂ (3 x 100mL). The combined organic layer was dried over MgSO₄, filtered and the filtrate was concentrated *in vacuo* to give a residue which was carried to the next step without further purification. Precursor 3a, Methyl (7-chloro-6-azaindol-3-yl)-oxoacetate: MS *m*/*z:* (M+H)⁺ calcd for C₁₀H₈ClN₂O₃: 239.02; found 238.97. HPLC retention time: 1.07 minutes (column A).

### Precursor 4a

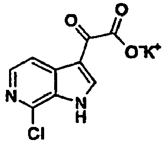

*Typical procedure of hydrolysis of ester:* Preparation of Potassium (7-chloro-6-azaindol-3-yl)-oxoacetate, Precursor 4a. Crude methyl (7-chloro-6-azaindol-3-yl)-oxoacetate, Precursor 3a, and an excess of K₂CO₃ (2 g) were dissolved in MeOH (20 mL) and H₂O (20 mL). After 8 h, the solution was concentrated and the residue was purified by silica gel column chromatography to provide 200 mg of Potassium (7-chloro-6-azaindol-3-yl)-oxoacetate. MS *m*/z: (M+H)⁺ of the corresponding acid was observed. Calc'd for C₉H₆ClN₂O₃ : 225.01; found 225.05. HPLC retention time: 0.83 minutes (column A).

### Precursor 5a

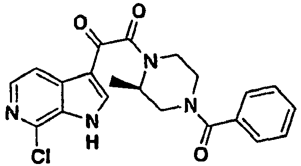

*Typical procedure for coupling piperazine derivative and azaindole acid:* Preparation of 1-benzoyl-3-(R)-methyl-4-[(7-chloro-6-azaindol-3-yl)-oxoacetyl]piperazine, Precursor 5. Potassium 7-chloro-6-azaindole 3-glyoxylate, Precursor 4a, (100 mg, 0.44 mmol), 3-(*R*)-methyl-1-benzoylpiperazine (107 mg, 0.44 mol), 3-(diethoxyphosphoryloxy)-1,2,3-benzotriazin-4(3*H*)-one (DEPBT) (101 mg, 0.44 mol) and Hunig's Base (diisopropylethylamine, 0.5 mL) were combined in 5 mL of DMF. The mixture was stirred at rt for 8 h. DMF was removed *via* evaporation at reduced pressure and the residue was purified using a Shimadzu automated preparative HPLC System to give 1-(benzoyl)-3-(R)-methyl-4-[(7-chloro-6-azaindol-3-yl)-oxoacetyl]-piperazine (70 mg, 39%). MS *m*/z: (M+H)⁺ Calc'd for C₂₁H₂₀ClN₄O₃: 411.12; Found 411.06. HPLC retention time: 1.32 minutes (column A).

### Precursor 5b

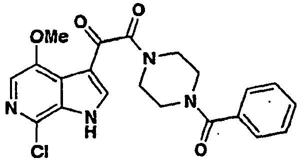

Precursor 5b, 1-benzoyl-4-[(7-chloro-4-methoxy-6-azaindol-3-yl)-oxoacetyl]piperazine was prepared by the same method as Precursor 5a starting from Potassium (7-chloro-4-methoxy-6-azaindol-3-yl)-oxoacetate, Precursor 4d, and 1-benzoylpiperazine. MS *m*/z: (M+H)⁺ calcd for C₂₁H₂₀ClN₄O₄: 427.12; found 427.12. HPLC retention time: 1.28 minutes (column A).

### Precursor 5i

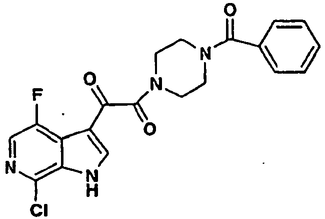

Addition of precursor 2d to a solution of aluminum trichloride in dichloromethane stirring at ambient temperature followed 30 minutes later with chloromethyl or chloroethyl oxalate (according to the method described for precursor 3a) provides either the methyl or ethyl ester, respectively. Hydrolysis with KOH (as in the standard hydrolysis procedure described for precursor 4a) provided potassium (7-chloro-4-fluoro-6-azaindol-3-yl)oxoacetate. Potassium (7-chloro-4-fluoro-6-azaindol-3-yl)oxoacetate was then reacted with 1-benzoyl piperazine in the presence of DEPBT under the standard conditions (as described for precursor 5a) to provide 1-benzoyl-4-[(4-fluoro-7-chloro-6-azaindol-3-yl)-oxoacetyl]piperazine, precursor 5i. ¹ H NMR (500 MHz, CD₃OD) δ 8.40 (s, 1H), 8.04 (s, 1H), 7.46 (bs, 5H), 3.80-3.50 (m, 8H); LC/MS (ES⁺) m/z (M+H)⁺ 415 observed; retention time 1.247 minutes; LC/MS method: YMC ODS-A C18 S7 3.0 x 50 mm column; Start %B = 0, Final %B = 100, Gradient time = 2 minutes; Flow rate = 5 mL/min; detector wavelength = 220 nm.

### EXAMPLE 1

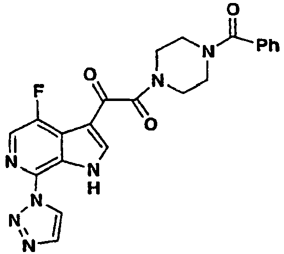

Example 1 was prepared from precursor 5i and 1,2,3-triazole following the procedure described above. ¹H NMR (500MHz, CDCl₃): 11.16 (bs, 1H); 8.75 (s, 1H); 8.37-8.37 (s, 1H); 8.15 (s, 1H); 7.92 (s, 1H); 7.43 (bs, 5H); 3.99-3.48 (m, 8H). LC/MS: (ES⁺) m/z (M+H)⁺ = 448. Rt = 1.28min.

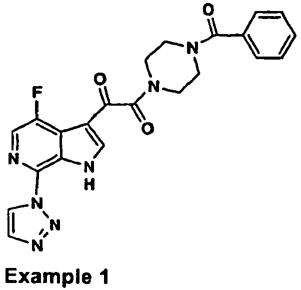

### Synthetic Experimental Procedures for best preparation of Example 1 (Scheme 1)

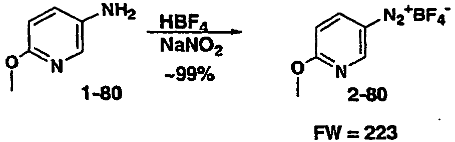

5-Amino 2 methoxypyridine (50g, 0.4mol ) was added to a stirring mixture of absolute ethanol (280 ml) and HBF₄ (48% in water, 172 ml) and cooled to 0°C. Sodium nitrite (129g) was dissolved in water (52 ml) and added portion-wise over 1h). The stirring was continued at 0°C for 2hr. The reaction mixture was diluted with ether (1L). The solid product was collected by filtration and washed with 500 ml of 50:50 EtOH/ether and subsequently several times with ether until the product was slightly pinkish in color. The pale pink solid 90g (∼100% yield) was kept in a dessicator over P₂O₅.

The same procedure was followed to perform the reaction on larger scale:
*(1) (200g, 1.6 mol); HBF₄ (688 ml); NaNO₂ (116 g); EtOH (1.12 L); H₂O (208 ml)*

The reaction was run 4 times (total 800 grams (1-80)). The product was dried over P₂O₅ for 48 hr. (only 24hr for first batch).

A total of 1,293 g of (2-80) was obtained, (91 % yield).

### Ref: J. Heterocyclic Chem., 10, 779, 1973 (for above reactions, including analytical data)

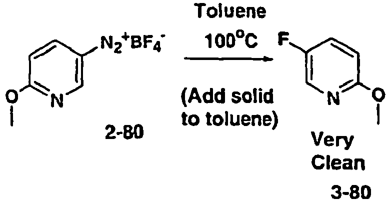

The decomposition of the diazonium salt was run in 3 batches of:
206g, 219g and 231g using 1.3L, 1.4L and 1.6L of anhydrous toluene respectively.

The toluene was preheated under nitrogen to 100°C (internal temperature) in a 2L 3-neck round bottom flask provided with a mechanical stirrer. The solid was added solid portion-wise via a scoop through a powder funnel which was attached to an adapter with slight outward positive nitrogen flow. During addition, the temperature was maintained between 99-102°C (set at 100°C) and stirred vigorously. Total addition time was 60 min. for the smaller two batches and 70 min. for the last one. After the addition was finished, each stirring reaction was heated at 110°C for 1hr. The heating mantle was removed and stirring was stopped. The reactions were allowed to stand for 2hr (ambient temp achieved). **Safety Note: The reaction contains BF3 so working with the reaction hot exposes vapors which caused skin irritation with some people. No incidents were noted at ambient temperature (6 different people).** The hot toluene from the reaction was poured into a 4L Erlenmeyer (a dark brown oil and residue remained in the flask). The residue was washed with 50 ml of toluene and poured into the original toluene extracts.

Add 1.5L of 1N NaOH to toluene layer, extract and wash with -100 ml of sat aq. NaCl.

Combine NaCl with NaOH layer, re-extract with 150 ml of toluene, wash with 50ml of sat NaCl.

Combine toluene layers.

Add 1L of 1N NaOH to residue in reaction flask and swirl to dissolve as much residue as possible then add 500ml) Et2O and pour into Erlenmeyer.

Add ∼500ml) more of 1 N NaOH to reaction flask and swirl ∼500ml of Et2O.

Combine dark Et2O and NaOH washings in erlenmyer flask.

Et2O/NaOH mixture was poured through powder funnel containing plug of glass wool to collect dark viscous solid. (Add ∼500ml more ether to wash) into 6L sep funnel.

Extract. Wash ether layer with ∼200ml of H₂O and then 100ml of sat NaCl.

Combine all washings with original NaOH aq. Layer and re-extract with 500ml) of ether. Wash with 100ml H₂O and 100ml of NaCl.

Combine ether extracts. Toluene and ether extracts were checked by LC/MS clean product.

The ether was concentrated on a rotovap and the residue was combined with the toluene extracts to make a homogeneous solution which is taken to next step as is.

The other two rxns were combined and worked up in the same way.

All aqueous layers were checked by LCMS = no product.

### Ref: J. Heterocyclic Chem., 10, 779, 1973 (for above reactions, including analytical data)

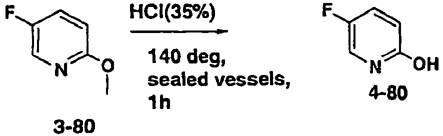

A total of 4.6L of toluene solution containing **3-80** was placed in several sealed tubes and treated with 900ml of 35% HCl at 145°C for 2hr. LC/MS showed no starting material, only 4. The toluene solution was decanted and discarded. The aqueous phase was washed with EtOAc and concentrated down to remove volatiles to afford a brown solid containing the desired fluoro-hydroxypyridine **4-80.**

A total of 244g of this solid was collected and taken to next step as is (it was not completely dry).

Note: We have subsequently run this by decanting the toluene layer first prior to heating to reduce volumes. Same reaction was carried out using HBr (48% in H2O) at 100°C for 6h with similar result to the literature procedure 49% yield.

### Ref: J. Heterocyclic Chem., 10, 779, 1973 (for above reactions, including analytical data)

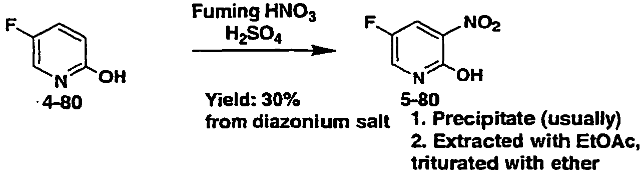

The solid from above containing **(4-80)** was divided in 4 batches and treated with H₂SO₄ and fuming HNO₃ as shown below. The amounts used were:

| | batch 1 | batch 2 | batch 3 | batch 4 |
|---|---|---|---|---|
| (1) | 25g | 54g | 75g | 90g |
| fuming HNO₃ | 20.8ml | 45ml | 62.4ml) | 75ml |
| H₂SO_{4 (for addition)} | 5.6ml+ | 12ml+ | 16.8ml+ | 20ml+ |
| (for soln) | 56ml | 120ml | 168ml | 200ml |

Compound **4-80** was dissolved in sulfuric acid (the larger amounts indicated above) at rt and then heated to 65°C. A preformed solution of fuming nitric acid and sulfuric acid (the smaller amount indicated above) was added dropwise. The temperature was kept between 65°C and 80°C (rxn is exothermic and although the bath is at 65°C, temperature goes higher, usually 75, sometimes 80°C). After the addition was complete, the reaction mixture was heated at 65°C for an additional hr. The reaction mixture was then cooled to rt and poured in a flask containing ice ) (20g of ice/gr compound, evolution of gas occurred). A solid precipitated out and it was collected by filtration (¹HNM" showed **4-80** and something else (discarded)).

The aqueous layer was extracted with AcOEt several times (3-5) and concentrated on a rotary evaporator under vacuum to afford a solid that was triturated with ether to afford 5-80 as a bright yellow solid. A total of 117g of desired product was collected in the first crop (27% yield from diazonium salt). A portion did not crystallize: this oil was triturated with MeOH and Et₂O to afford 3.6g of **5-80**; another precipitation from the mother liquid afforded an additional 6.23g of the desired product **5-80**

Total:117.0+3.6+6.23 = 126.83. 30.4%). Yield for 3 steps (decomposition of diazonium salt; deprotection and nitration).

Analytical data from Notebook: 53877-115: ¹HNMR(δ, MeOD): 8.56-8.27 (dd, J= 7.5, 3.3 Hz, 1H), 8.01 (d, J=3.3 Hz, 1H); LC/MS(M+1)⁺= 158.9; rt = 0.15 min.

Note: A portion of the aqueous acidic solution was taken and neutralized with Na₂CO₃ until effervescence stopped and then it was extracted with AcOEt ⇒ A different product was obtained. No desired product in these extracts.

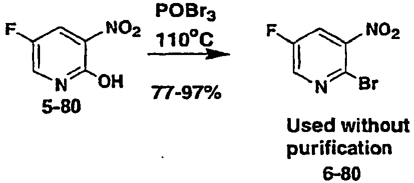

A total of 117g of **5-80** was divided in 4 batches of 30g x 3 and 27g x 1 and treated with POBr₃ (3 equiv.; 163g x 3 and 155 g x 1) and a catalytic amount of DMF (15 ml) at rt (DMF was added carefully ⇒ gas evolution). After 5 min. at room temperature, the solutions were heated at 110°C for 3hr. LC/MS showed starting material had been consumed. The reaction mixtures were allowed to cool to rt. The reaction flasks were placed in an ice bath; and then ice was added very slowly and carefully portionwise into the flask, gas evolution was due to HBr formation; the liquid and black solid that formed was poured into a beaker with ice. EtOAc was added and the mixture was then extracted several times with EtOAc. The organic layer was washed with saturated aq. NaHCO₃; H₂O and brine; dried over Na₂SO₄ and filtered. The product was dried in the pump overnight to provide 123g of **6-80** as a brown solid (77% yield).

Note: Reaction is completed within 1h.

¹HNMR(δ, CDCl₃):8.52 (m, 1H), 7.93 (m, 1H).

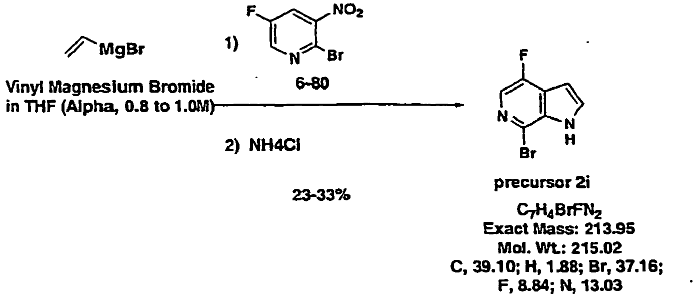

800 ml of vinyl magnesium bromide (1M in THF, Aldrich) was cooled below -60°C with vigorous stirring under N₂. 2-bromo-5-fluoro-3-nitro pyridine (43.3g, 0.196 mol) in 200ml THF was added dropwise via addition funnel at such a rate that the temp was kept below -60°C. This took - 1.25 hr. The reaction mixture was warmed to -40 to -50°C and stirred for 1 hr more. Then 1L of saturated aqueous NH₄Cl was added slowly and cautiously. At first, foaming occurred and considerable solid was present, but this essentially dissolved as the addition was completed and the material warmed to rt. The layers were separated and the aqueous layer extracted 3 times with ethyl acetate. The organic extracts were washed with brine, dried over Na₂SO₄, filtered and concentrated to afford - 50g of a black gummy solid. HPLC indicated 57-58% product. To this was added CH₂Cl₂ and the solid was collected by filtration and washed with CH₂Cl₂ to afford 12.5g of product as a brown solid. The reaction was repeated on exactly the same scale and worked up in the same manner. From CH₂Cl₂ trituration there was obtained 12.4g of **Precursor 2i** (HPLC ∼ 97% pure). The crude was recovered and allowed to stand in dichloromethane. Upon standing 3.6g of additional product separated and was recovered by filtration.

Total yield = 29.5g (35%).

¹HNMR(δ, CDCl₃): 8.69(bs, 1H), 7.92 (d, J= 1.8 Hz, 1H), 7.41 (m, 1H), 6.77 (m,1H); LC/MS(M+1)⁺= 216.-217.9; rt= 1.43 min.

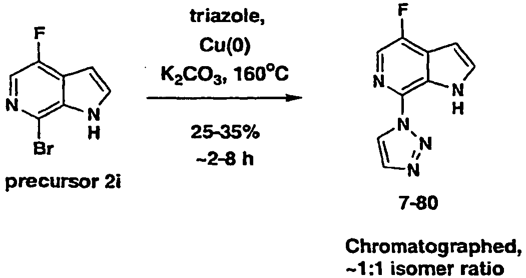

Reaction was carried in a 250ml flask (foaming occurred upon heating and the big size flask is more convenient). A mixture of precursor **2i** (3g, 13.95mmol), 1,2,3-triazole (15g, 217.6 mmol, 15eq), K₂CO₃ (1.9g, 13.95mmol), 1eq) and Cu(0)(0.9g, 13.9mmol, 1eq) was heated at 160°C for 7 hr (from rt to 160°C total 7 hr) under N₂ (depending on the Cu(0) lot, reaction time may vary from 2hr to 7hr). The resulting mixture was diluted with MeOH, filtered through filter paper (to remove the copper). Washed with MeOH (20 ml) and water (30 ml).

The filtrate was concentrated (remove solvent in rotovap) and diluted with ethylacetate. The aqueous layer was extracted with ethylacetate. The combined organic layer was dried over sodium sulfate, filtered and concentrated. The residue was dissolved in MeOH (20 ml), **7-80** (750mg) crystallized from the methanol as a white solid and was collected by filtration. (Slow gradient volume, silica gel hex /AcOEt (0→18%) of the mother liquids usually affords 5-10% more of **7-80**.

¹HNMR (δ, CDCl₃): 10.47 (bs, 1H), 8.76 (s, 1H), 7.94 (s, 1H), 7.89 (s, 1H), 7.53 (m, 1 H), 6.78 (m, 1H); LCMS(M+1)⁺= 204; rt = 1.29 min.

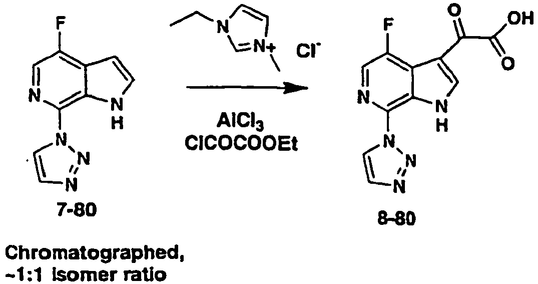

Ethyl methylimidazolium chloride (4.3g, 29.6 mmol, 3eq) was placed in a 250ml flask. AlCl₃ (11.8g, 88.6mmol, 9eq) was added into the flask in one portion. A liquid suspension was formed (some of AlCl₃ remained as solid). After stirring for 5-10 min. compound (1) (2.0g, 9.85mmol) was added in one portion followed by slow addition (via a syringe) of ethyl chlorooxalacetate (3.3 ml, 29.6 mmol, 3eq). The reaction was stirred at room temperature for 20 hr. LCMS indicated compound 8-80:compound 7-80 = 6:2. (Compound I has strong UV absorption) The reaction was quenched by carefully adding ice water (∼75 ml) at 0°C. A yellow solid precipitated at this point. The resulting suspension was filtered and the solid was washed with water. MeOH and ethyl acetate (to remove unreacted SM) and the solid was dried in air. (LCMS purity 70% ∼ 80%) 2g of solid containing **8-80** was obtained and taken to the next step without further purification. LCMS(M+1)⁺= 276; rt =0.97 min.

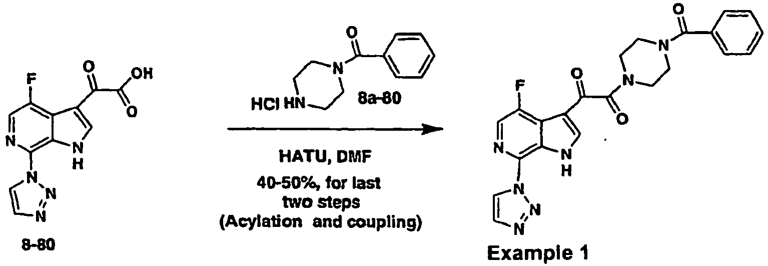

A mixture of compound **8-80** (4.9g,17.8 mmol) & N-benzoylpiperazine hydrochloride **8a-80** (HCl salt; 6.0g, 26.7mmol, 1.5eq) in DMF (30 ml) was stirred at RT overnight (16 hr). A slurry was formed. An additional 20ml of DMF was added into the slurry. Then HATU (12.2g, 26.7mmol, 1.5eq) was added followed by DMAP (4.3g, 35.6 mmol, 2eq). The reaction mixture was stirred for 30 min. LCMS indicated the starting material **8-80** was completely converted to product (EXAMPLE 216). The resulting mixture was filtered and the solid washed with water. The filtrate was concentrated in vacuo. Water was added to the residue and the solid was collected by filtration. The solids were combined and washed with water, MeOH and EtOAc. Then the solid was dried in air. LCMS & HPLC showed BMS-585248, >99% pure. The solid product was further purified by precipitation and crystallization in 5∼10% CH₃OH/CHCl₃.

### Purification of Example 1

Crude compound of Example 1 obtained as above (15.3g) was dissolved in 10% MeOH/CHCl₃ (600 ml). A light brown suspension was formed, filtered through filter paper and washed with MeOH a twice. The brownish solid was discarded (∼1.2g). Example 216 was crystallized in the filtrate, the solid was collected by filtration and the white solid was dried in air. The filtrate was used to repeat the crystallization several times. The solid obtained from each filtration was analyzed by HPLC. All the pure fractions were combined. The not so pure fractions were resubjected to crystallization with MeOH & CHCl₃. A total of 12.7g of Example 1 was obtained from recrystallization and precipitation. The mother liquid was concentrated and purified on silica gel column (EtOAc, then CHCl₃/MeOH (0-2%)) to provide 506mg of product) as a white solid.

¹HNMR (d, DMSO) 13.1 (bs, 1H), 9.0 (s, 1H), 8.4 (s, 1H), 8.3 (s, 1H), 8.2 (s, 1H), 7.4 (bs, 5H), 3.7 (bs, 4H), 3.5 (bs, 4H); MS m/z 448 (MH). Anal: Calc for C₂₂H₁₈FN₇O₃; C 59.05, H 4.05, N 21.91, F 4.24. Found; C 57.28, H 4.14, N 21.22; F 4.07%.

Scheme 2 is a preferred method for the preparation of compound Example 2.

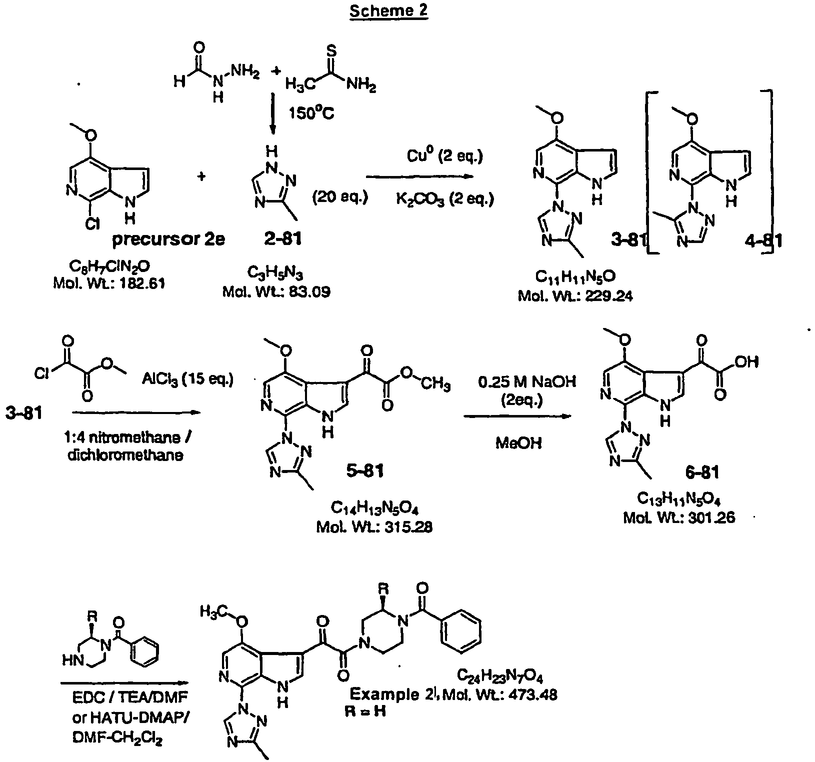

### Preparation of 3-methyl-1,2,4-triazole (2-81)

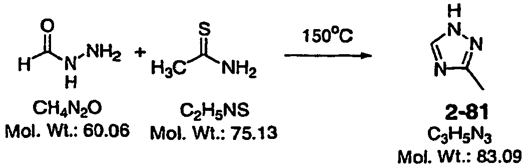

**Procedure:** A solid mixture of formic hydrazide (68 g, 1.13 mol) and thioacetamide (85 g, 1.13 mol) in a 500mL-RBF was heated with stirring at 150°C (oil bath temp.) for 1.5 hrs with a gentle stream of nitrogen, removing H₂S and water (about 18 mL of liquid collected) formed during the reaction. The reaction mixture was distilled under reduced pressure, collecting 60.3 g (0.726 mol, Y. 63.3%) of the title compound at 102°C / 0.35-1 mmHg as white solid after removing a liquid forerun. : ¹H NMR (CDCl₃) δppm 2.51 (3H, s, 3-Me), 8.03 (1H, s, 5-H), 9.5 (1H, br, NH); TLC Rf (10% MeOH/CH₂Cl₂) = 0.3 (phosphomolybdate-charring, white spot). Reference: Vanek, T.; Velkova, V.; Gut, Jiri *Coll. Czech. Chem. Comm.* **1985**, *49*, 2492.

### Preparation of 3-81

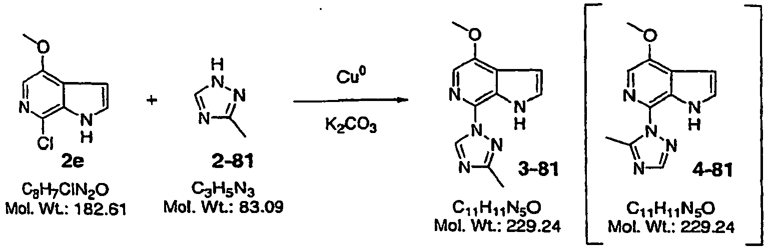

**Procedure:** A 500 mL round bottom flask was loaded with 4-methoxy-7-chloro-6-azaindole **precursor 2e** (9.1 g, 50 mmol; dried *in vacuo),* potassium carbonate (13.8 g, 100 mmol, 2 eq.), copper powder (6.35 g, 100 mmol, 2 eq.), and 3-methyl-1,2,4-triazole (83 g, 1.0 mol, 20 eq.). The solid mixture was heated to melt at 170-175°C (external oil bath temperature) under gentle stream of anhydrous nitrogen for 12 h, by which time HPLC analysis indicates the amount of the peak for the starting material becomes 5-30% and the desired product peak becomes about 45% with isomeric byproduct peak becomes 15%. As the reaction mixture cools, MeOH (150 mL) was added slowly to the stirred warm mixture. Upon cooling, the insoluble material (copper powder) was filtered through a Celite pad, and rinsed with methanol. The filtrate was concentrated *in vacuo* to a thick paste which was diluted with water (1 L) and extracted with EtOAc (3x150mL). The EtOAc extracts were dried (MgSO₄), filtered and concentrated to obtain about 8 g of crude residue which was crystallized by dissolving in hot CH₃CN (50 mL), followed by diluting with water (100 mL) and cooling at 0°C to collect 1.45 g (12.7%) of the title compound as white solid. The filtrate was purified by C-18 reverse phase silica gel (YMC ODS-A 75 µm) eluted with 15-30% CH₃CN/H₂O. Appropriate fractions were combined and the aqueous solution after removing CH₃CN by rotary evaporator was lyophilized to give additional 1.15 g of the title compound **3-81**. The crude aqueous layer was further extracted with EtOAc several times. The ethyl acetate extracts were dried (MgSO₄), filtered, concentrated, and crystallized from MeOH to give additional 200 mg of the title compound **3-81**. The total yield: 2.8 g (12.2 mmol, Y. 24.5%); MS m/z 230 (MH), HRMS (ESI) m/z calcd for C₁₁H₁₂N₅O (M+H), 230.1042, found 230.1038 (Δ - 1.7 ppm); ¹H NMR (CDCl₃) δppm 2.54 (3H, s, CH₃), 4.05 (3H, s, OCH₃), 6.73 (1H, s, H-3), 7.40 (1H, s, H-2), 7.56 (1H, s, H-5), 9.15 (1H, s, triazole-H-5); ¹³C NMR (CDCl₃, 125.7 MHz) δ ppm 14.2 (triazole-Me), 56.3 (OMe), 100.5 (C-3), 116.9 (C-5), 123.5, 127.2, 127.5 (C-2), 129.5 (C-7), 141.2 (C-5'), 149.5 (C-4), 161.8 (C-3'); Anal. Calcd for C₁₁H₁₁N₅O: C 57.63, H 4.83, N 30.55, found C 57.37, H 4.64, N 30.68.

The structure was confirmed by a single X-ray crystallographic analysis using crystals obtained from C-18 column fractions. A portion of C-18 column fractions containing a mixture of the desired 3-methyl-1,2,4-triazolyl analog **3-81** and isomeric 5-methyl-1,2,4-triazolyl analog **4-81** was further purified by C-18 reverse phase column eluting with 8-10% CH₃CN/H₂O. Appropriate fractions were extracted with CH₂Cl₂, and slow evaporation of the solvent gave crystalline material of the isomeric 7-(5-methyl-1,2,4-triazolyl)-4-methoxy-6-azaindole (**4-81**): MS m/z 230 (MH), ¹H NMR (CDCl₃) δppm 3.05 (3H, s, CH₃), 4.07 (3H, s, OCH₃), 6.74 (1H, q, J=2.4, H-2), 7.37 (1H, t, J=2.4, H-3), 7.65 (1H, s, H-5), 8.07 (1H, s, triazole-H-3). The structure was confirmed by a single X-ray crystallographic analysis.

### Preparation of 5-81

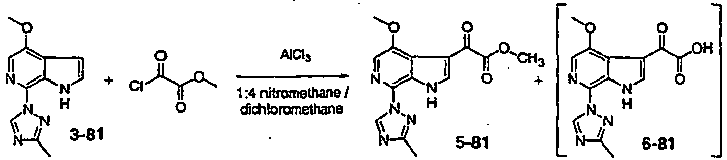

**Procedure:** AlCl₃ (40 g, 0.3 mol, 15 eq.) was dissolved in a solution of CH₂Cl₂ (100 mL) and nitromethane (20 mL) under dry nitrogen. To this solution was added compound **3-81** (4.58 g, 0.02 mol) under stirring and under N₂, followed by methyl chlorooxoacetate (9.8 g, 0.08 mol, 4 eq.). The mixture was stirred under N₂ at room temperature for 1.5 h. The mixture was added drop-wise to a cold and stirred solution of 20% aqueous ammonium acetate solution (750 mL). The mixture was stirred for 20 min and the resultant precipitate was filtered, washed thoroughly with water and dried *in vacuo* to obtain 4.7 g (0.015 mol, Y. 75%) of the title compound 5-81 as white solid: MS m/z 316 (MH); HRMS (ESI) m/z calcd for C₁₄H₁₄N₅O₄ (M+H), 316.1046; found 316.1041 (Δ -1.6 ppm); ¹H NMR (CDCl₃, 500 MHz) δppm 2.58 (3H, s, CH₃), 3.96 (3H, s, OCH₃), 4.05 (3H, s, OCH₃), 7.76 (1H, s, H-5), 8.34 (1H, d, J=3Hz, H-2), 9.15 (1H, s, triazole-H-5), 11.0 (1H, brs, NH). More title compound 5-81 and hydrolyzed acid 6-81 can be obtained from the filtrate by acid-base extraction with EtOAc.

### Preparation of 6-81

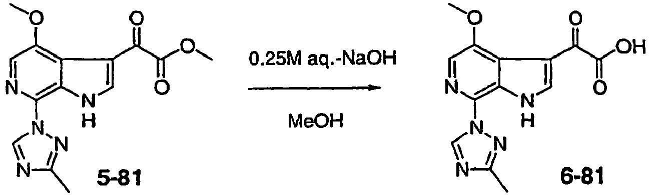

**Procedure:** To a suspension of the methyl ester **5-81** (2.2 g, 7.0 mmol) in MeOH (50 mL) was added 0.25M NaOH solution in water (56 mL, 14 mmol, 2 eq.) at room temperature and the mixture stirred for 15 min by which time HPLC indicated the hydrolysis was complete. The mixture was concentrated *in vacuo* quickly to remove MeOH, and to the residual solution was added water (100 mL) and 1N HCl (14 mL) with stirring to neutralize the mixture. The resultant fine precipitate was filtered, washed with water and dried *in vacuo* to obtain 1.98 g ( 6.58 mmol, Y. 94%) of the title compound **6-81** as off-white solid: MS m/z 302 (MH); ¹H NMR (DMSO-*d₆*, 500 MHz) δ ppm 2.50 (3H, s, overlapped with DMSO peaks), 3.98 (3H, s, CH₃O), 7.87 (1H, s, H-5), 8.29 (1H, d, J=3.5Hz, H-2), 9.25 (1H, s, triazole-H-5), 12.37 (1H, s, NH).

Alternative procedure: To a suspension of the methyl ester **5-81** (10.7 g, 34 mmol) in MeOH (150 mL) was added 0.25M NaOH solution in water (272 mL, 68 mmol, 2 eq.) at room temperature and the mixture stirred for 20 min by which time HPLC indicated the hydrolysis was complete. The mixture was concentrated *in vacuo* quickly to remove MeOH, and the residual solution was extracted with EtOAc to remove any neutral impurities. To the aqueous phase was added 1N HCl (68 mL, 68 mmol) to neutralize the product. The resultant mixture was frozen and lyophilized to obtain 14.1 g ( 33.7 mmol, Y. 99.2%) of the title compound **6-81,** containing 2 mole equivalents of NaCl as off-white solid. This material was used in the subsequent reaction without further purification. The sodium salt of the title compound **6-81** was obtained by C-18 reverse phase column chromatography after sodium bicarbonate treatment: HPLC >97% (AP, uv at 254nm); HRMS (Na salt, ESI ⁻) m/z calcd for C₁₃H₁₀N₅O₄ (M-H), 300.0733; found 300.0724 (Δ -3 ppm); ¹H NMR (Na salt, DMSO-d₆, 500 MHz) δ ppm 2.37 (3H, s, Me), 3.83 (3H, s, CH₃O), 7.56 (1H, s, H-5), 8.03 (1H, s, H-2), 9.32 (1H, s, triazole-H-5); ¹³C NMR (Na salt, DMSO-*d₆*, 125.7 MHz) δ ppm 13.8 (triazole-Me), 57.2 (OMe), 114.8 (C-3), 120.0 (C-5), 125.1, 143.5 (C-5'), 149.8 (C-4), 160.0 (C-3'), 171.7,191.3.

### Preparation of Example 2

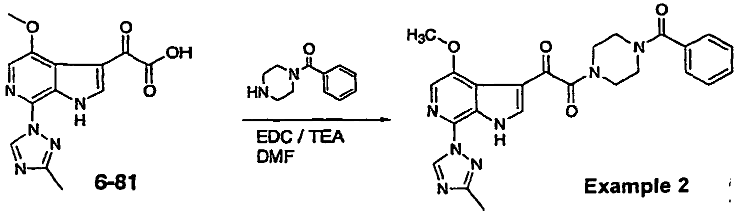

Procedure: To a solution of the acid **6-81** (3.01 g, 10 mmol) and benzoylpiperazine hydrochloride (3.39 g, 15 mmol) in DMF (50 mL) was added triethylamine (10.1 g, 100 mmol, 10 eq.), followed by 1-[3-(dimethylamino)propyl]-3-ethylcarbodiimide hydrochloride (EDC; 5.75 g, 30 mmol) under N₂ and the mixture stirred at room temperature for 22 h after sonication and at 40°C for 2 h. The mixture was concentrated *in vacuo* to remove DMF and TEA, and to the residual solution was added water (200 mL) under stirring and sonication. The precipitates formed were collected, washed with water and dried *in vacuo* to obtain 2.8 g (5.9 mmol, Y. 59%) of the title compound **Example 2** as off-white solid. The filtrate was extracted with CH₂Cl₂ (x2). The CH₂Cl₂ extracts were dried (Na₂SO₄), filtered and concentrated to gum which was triturated with Et₂O to obtain a solid. This solid was suspended and triturated with MeOH to obtain 400 mg of the title compound **Example 2** as off-white solid. Total yield: 3.2 g (6.8 mmol, Y. 68%): MS m/z 474 (MH); HRMS (ESI) m/z calcd for C₂₄H₂₄N₇O₄ (M+H) 474.1890, found 474.1884 (Δ - 1.2 ppm); ¹H NMR (DMSO-*d6*) δ ppm 2.50 (3H, s, overlapped with DMSO peaks), 3.43 (4H, br, CH₂N), 3.68 (4H, br, CH₂N), 3.99 (3H, s, CH₃O), 7.46 (5H, br. s, Ar-Hs), 7.88 (1H, s, indole-H-5), 8.25 (1H, s, indole-H-2), 9.25 (1H, s, triazole-H-5), 12.40 (1H, s, NH); ¹³C-NMR (DMSO-d6) δ ppm 13.78, 40.58, 45.11, 56.78, 114.11, 120.95, 122.71, 123.60, 126.98, 128.34, 129.6, 135.43, 138.52, 142.10, 149.15, 161.29, 166.17, 169.22, 185.42; UV (MeOH) λmax 233.6 nm (ε 3.43x10⁴), 314.9 nm (ε 1.73x10⁴); Anal: Calc for C₂₄H₂₄N₇O₄.1/5H₂O; C 60.42, H 4.94, N 20.55, Found; C 60.42, H 5.03, N 20.65; KF (H₂O) 0.75%.

This reaction can also be performed by use of HATU and DMAP to provide more consistent yield of the title compound: To a suspension of the acid 6-81 (15.6 mmol) and HATU [O-(7-azabenzotriazol-1-yl)-N,N,N',N'-tetramethyluronium hexafluorophos phonate] (8.90 g, 23.4 mmol; 1.5 eq.) in DMF (60 mL) and CH₂Cl₂ (60 mL) was added a mixture of DMAP (5.72 g, 46.8 mmol, 3 eq.) and benzoylpiperazine hydrochloride (5.30 g, 23.4 mmol; 1.5 eq.) in DMF (60 mL) at room temperature and the mixture was stirred under nitrogen atmosphere for 4 hrs. The mixture was concentrated *in vacuo* to remove CH₂Cl₂ and most of DMF, and to the residual solution was added water under stirring and sonication. The precipitates formed were collected, washed with water and dried in *vacuo* to obtain 5.38 g (11.4 mmol, Y. 72.8%) of the title compound **Example 2** as off-white solid: HPLC >95% (AP, uv at 254nm).

### Alternate Preparation of Example 2

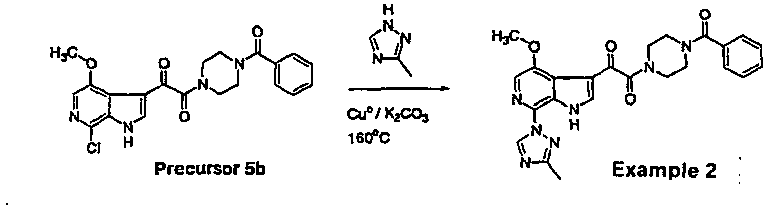

A. mixture of compound **precursor 5b** (150 mg, 0.35 mmol), 3-methyl-1 ,2,4-triazole (581 mg, 7 mmol; 20 eq.; prepared by the method described in Coll. Czech. Chem. Comm. 1985, 49, 2492), copper powder (45 mg, 0.7 mmol; 2 eq.), potassium carbonate (97 mg, 0.7 mmol; 2 eq.) was flushed with anhydrous nitrogen and heated in a sealed tube at 160°C for 11 h. Upon cooling, to the mixture was added MeOH, and the insoluble material was filtered. The filtrate was concentrated in *vacuo* and purified by C-18 reverse phase column (Prep. System eluting with MeOH-water containing 0.1% TFA) to obtain 19 mg (0.040 mmol), Y. 11%) of the title compound **Example 2** as amorphous powder (TFA salt): MS m/e 474 (MH); ¹H NMR (DMSO-d6) δ ppm 2.50 (3H, s, overlapped with DMSO peaks), 3.44 (4H, br, CH₂N), 3.68 (4H, br, CH₂N), 4.00 (3H, s, CH₃O), 7.46 (5H, br. s, Ar-Hs), 7.89 (1H, s), 8.25 (1H, s), 9.24 (1H, s), 12.41 (1H, s, NH).

### Biology

- "µM" means micromolar;
- "mL:' means milliliter,
- "µl" means microliter,
- "mg" means milligram;

The materials and experimental procedures used to obtain the results reported are described below.

Cells:
- Virus production-Human embryonic Kidney cell line, 293, propagated in Dulbecco's Modified Eagle Medium (Life Technologies, Gaithersburg, MD) containing 10% fetal Bovine serum (FBS, Sigma, St..Louis , MO).
- Virus infection- Human epithelial cell line, HeLa, expressing the HIV-1 receptors CD4 and CCR5 was propagated in Dulbecco's Modified Eagle Medium (Life Technologies, Gaithersburg, MD) containing 10% fetal Bovine serum (FBS, Sigma, St. Louis , MO) and supplemented with 0.2 mg/mL Geneticin (Life Technologies, Gaithersburg, MD) and 0.4 mg/mL Zeocin (Invitrogen, Carlsbad, CA).

Virus-Single-round infectious reporter virus was produced by co-transfecting human embryonic Kidney 293 cells with an HIV-1 envelope DNA expression vector and a proviral cDNA containing an envelope deletion mutation and the luciferase reporter gene inserted in place of HIV-1 nef sequences (Chen et al, Ref. 41). Transfections were performed using lipofectAMINE PLUS reagent as described by the manufacturer (Life Technologies, Gaithersburg, MD).

### Experiment

1. Compound was added to HeLa CD4 CCR5 cells plated in 96 well plates at a cell density of 5 X 10⁴ cells per well in 100 µl Dulbecco's Modified Eagle Medium containing 10 % fetal Bovine serum at a concentration of <20 µM.
2. 100 µl of single-round infectious reporter virus in Dulbecco's Modified Eagle Medium was then added to the plated cells and compound at an approximate multiplicity of infection (MOI) of 0.01, resulting in a final volume of 200 µl per well and a final compound concentration of <10 µM.
3. Samples were harvested 72 h after infection.
4. Viral infection was monitored by measuring luciferase expression from viral DNA in the infected cells using a luciferase reporter gene assay kit (Roche Molecular Biochemicals, Indianapolis, IN). Infected cell supernatants were removed and 50 µl of Dulbecco's Modified Eagle Medium (without phenol red) and 50 µl of luciferase assay reagent reconstituted as described by the manufacturer (Roche Molecular Biochemicals, Indianapolis, IN) was added per well. Luciferase activity was then quantified by measuring luminescence using a Wallac microbeta scintillation counter.
5. The percent inhibition for each compound was calculated by quantifying the level of luciferase expression in cells infected in the presence of each compound as a percentage of that observed for cells infected in the absence of compound and subtracting such a determined value from 100.
6. An EC₅₀ provides a method for comparing the antiviral potency of the compounds of this invention. The effective concentration for fifty percent inhibition (EC₅₀) was calculated with the Microsoft Excel Xlfit curve fitting software. For each compound, curves were generated from percent inhibition calculated at 10 different concentrations by using a four paramenter logistic model (model 205). The EC₅₀ data for the compounds is shown in Tables 2-4. Table 1 is the key for the data in Tables 2-4.

Cytoxicity assays were conducted with the same HeLa using methodology well known in the art. This method has been described in the literature (S Weislow, R Kiser, DL Fine, J Bader, RH Shoemaker and MR Boyd: New soluble-formazan assay for HIV-1 cytopathic effects: application to high-flux screening of synthetic and natural products for AIDS-antiviral activity. Journal of the National Cancer Institute. 81(8):577-586, 1989.

Cells were incubated in the presence of drug for six days, after which cell viability was measured using a dye reduction assay (MTT) and determined as a CC50. This assay measures the intracellular reducing activity present in actively respiring cells.

### Results

**Table 1. Biological Data Key for EC₅₀s**

| Compounds* with EC₅₀s >5µM | Compounds with EC₅₀s >1 µM but <5µM | Compounds with EC50 >50nM but not yet tested at higher concentrations | Compounds with EC50 < 1 µM |
|---|---|---|---|
| Group C | Group B | Group A' | Group A |

The compounds of examples 1 and 2 belong to Group A.

**Metabolic Stability Studies of compounds in Liver Microsomes**. The metabolic stability of compounds were investigated in pooled liver microsomes from humans. The human liver microsomes were obtained from BD Gentest (Lot #16, Woburn, MA) with a protein concentration of 20 mg/ml and a total cytochrome P450 (CYP) concentration of 0.55 nmol/mg protein.

A stock solution of drug was prepared in acetonitrile at 1 mM. An aliquot of the stock solution was added to the incubation media to give a final concentration of 3 µM of drug, and the acetonitrile concentration not exceeding 1 % in the incubation. The incubation media consisted of potassium phosphate buffer (0.1 M, pH 7.4), liver microsomes (final concentration 0.9 mg/ml), magnesium chloride (0.033 mM), and a NADPH-regenerating system. The cofactors of the NADPH-regenerating system consisted of NADPH (final concentration 0.425 mg/ml), glucose-6-pbosphate (final concentration 0.512 mg/ml), and glucose-6-phosphate dehydrogenase (final concentration 0.6 unitiml). The test compound was pre-incubated in the media for 2 min. The reaction was initiated by the addition of the cofactors. The incubation was carried out at 37°C for 10 min. The reaction was terminated by drawing an aliquot of 100 µL from the incubation and adding into 200 µL of acetonitrile containing a reference compound as an external analytical standard. Following vortex-mixing and centrifugation, an aliquot of 10 µL of the supernatant was analyzed by LC/MS.

GUIDELINES can be used to categorized test substances as low, intermediate or highly cleared compounds.

| Rate (nmol/inin/mg) | Clearance Estimate |
|---|---|
| 0-0.100 | Low |
| 0.101 - 0.200 | Intermediate |
| 0.201 - 0.300 | High |

### Rat Pharmacokinetic Studies:

For the IV and PO pharmacokinetic studies of compounds in rats, the compound was dissolved in PEG-400/ethanol (90/10) as a solution.

**Rat.** Male Sprague-Dawley rats (300-350 g, Hilltop Lab Animals, Inc., Scottdale, PA) with cannulas implanted in the jugular vein were used. The rats were fasted overnight in the PO pharmacokinetic studies. Blood samples of 0.3 ml were collected from the jugular vein in EDTA-containing microtainer tubes (Becton Dickinson, Franklin Lakes, NJ), and centrifuged to separate plasma.

In the IV study, the test compound was delivered at 1 mg/kg as a bolus over 0.5 min (n = 3). Serial blood samples were collected before dosing and 2, 10, 15, 30, 45, 60, 120, 240, 360, 480, and 1440 min after dosing.

In the PO study of the test compound, the rats (n = 3) received an oral dose of 5 mg/kg of BMS-585248. Serial blood samples were collected before dosing and 15, 30, 45, 60, 120, 240, 360, 480, and 1440 min after dosing.

**Quantitation of Compounds in Plasma**. Aliquots of plasma samples from rat, studies were prepared for analysis by precipitating plasma proteins with two volumes of acetonitrile containing an internal standard of a similar compound. The resulting supernates were separated from the precipitated proteins by centrifugation for 10 minutes and transferred to autosampler vials. Samples were either prepared manually, or with the use of the Tomtec automated liquid handler.. An aliquot of 5 µL was injected for analysis.

The HPLC system consisted of two Shimadzu LC10AD pumps (Columbia, Ivy), a Shimadzu SIL-HTC autosampler (Columbia, MD), and a Hewlett Packard Series 1100 column compartment (Palo Alto, CA). The column was a YMC Pro C18 (2.0 x 50 mm, 3 µm particles, Waters Co., Milford, MA), maintained at 60°C and a flow rate of 0.3 ml/min. The mobile phase consisted of 10 mM ammonium formate and 0.1 % formic acid in water (A) and 100% 10 mM ammonium formate and 0.1% formic acid in methanol (B). The initial mobile phase composition was 95% A. After sample injection, the mobile phase was changed to 15% A/85% B over 2 minutes and held at that composition for an additional 1 minute. The mobile phase was then returned to initial conditions and the column re-equilibrated for 1 minute. Total analysis time was 4 minutes.

The HPLC was interfaced to a Micromass Quattro LC. Ultra high purity nitrogen was used as the nebulizing and desolvation gas at flow rates of 100 L/hr for nebulization and 1100 L/hr for desolvation. The desolvation temperature was 300°C and the source temperature was 150°C. Data acquisition utilized selected reaction monitoring (SRM). Ions representing the (M+H)⁺ species for the compound and the internal standard were selected in MS 1 and collisionally dissociated with argon at a pressure of 2 x 10⁻³ torr to form specific product ions which were subsequently monitored by MS2.

The compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a compound of the invention and its pharmaceutical composition for treating viral infections such as HIV infection and AIDS. The treatment involves administering to a patient in need of such treatment a pharmaceutical composition comprising a pharmaceutical carrier and a therapeutically-effective amount of a compound of the present invention.

The pharmaceutical composition may be in the form of orally-administrable suspensions or tablets; nasal sprays, sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The disclosure covers isomers, diasteroisomers, stereoisomers, and enantiomers of the depicted formulas when one or more asymmetric carbons are present in the molecules. An asymmetric carbon is one in which the carbon is attached to four different substitutions. The disclosure covers isomers or a single enantiomer especially when one enantiomer displays superior properties. Enantiomers differ from one another in that the spacial arrangement of the substituents around the chiral centers of the asymmetric carbons result in each molecule being a nonsuperimposable mirror image of the other. The configuration of the substituents around an asymmetric carbon are defined unambiguously as either (R) which is a standard representation which stands for Latin rectus, right or (S) which is the standard representation for Latin sinister, left in the Cahn-Ingold-Prelog nomenclature system which has been in use since the 1960s. Standard rules for defining the configuration of these centers are found in any basic organic chemistry textbook. For this disclosure and based on initial examples, when W contains a single methyl group as depicted below, when the carbon bearing the methyl group is in the (R) configuration it may show a potency advantage over the (S) enantiomer. Occasionally the (R)-methyl piperazine may show a potency advantage over the unsubstituted piperazine. These observations are compound specific effect and are not always present. The unsubstituted piperazine and (S) enantiomers are still potent antiviral compounds despite occasionally being less potent than the (R) enantiomer.

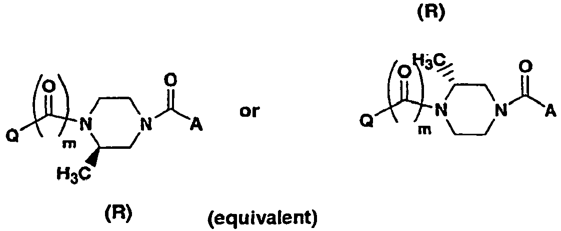

When the configuration of a methyl piperazine shown as below is (R) the methyl group may improve the metabolic stability of the adjacent amide as compared to the (S) methyl piperazine or the unsubstituted piperazine. However, the metabolic stability of the amide bond is compound specific and a methyl substituent is not necessarily required for optimal properties.

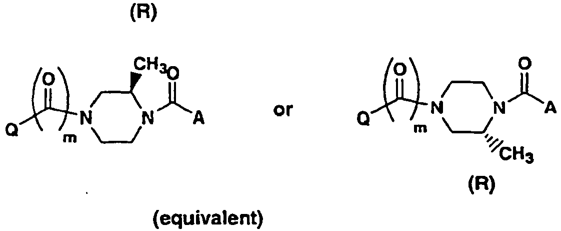

It has now also been surprisingly found that the compounds of the invention are particularly effective for inhibiting HIV. This is discussed more fully below.

The effective treatment of HIV and other viruses requires compounds that are potent inhibitors of the virus, are selective for the virus, and have the properties which allow them to safely achieve and maintain plasma level concentrations which are a maximum number multiples above the concentration required to minimally inhibit the virus. Higher exposures suppress viral replication and reduced rates of replication mean that strains of virus with resistance to the drug treatment will develop at a slower rate. Potent drugs exhibit equivalent activity from a lower concentration or lower dose than that needed to achieve the same effect from a less potent drug. Drugs which intrinsically produce higher exposures from an equivalent dose in animal models or patients (as determined by pharmacokinetic measurements such as AUC (the sum of the concentration of drug over a particular time), Cmax, or Cmin will also provide greater benefit to the patient. Drugs which have higher stability in the presence of metabolizing pathways and enzymes will maintain their concentrations longer and thus require less frequent dosing or dosing of smaller quantities. In animals or people the rate of clearance is a frequently measured parameter to assess this property but mean retention time is also used. For accuracy, the determined measure of viral inhibition is an EC50; but the minimum plasma concentrations which should be maintained in a patient is generally believed to be at least four or five fold higher. Thus the antiviral or anti HIV drug candidates which will be most likely to provide maximum benefits in patients and those that preclinical research programs strive to identify will exhibit 1) maximum potency 2) no general cytotoxicity vs the cell line used for the assay 3) low predicted rates of metabolism in human based on *in vitro* models 4) high exposure after oral dosing. Many other properties of potential drug candidates are evaluated in order to determine which compounds will have the best chance of showing optimal utility in human patients but the compounds of this invention were evaluated initially in part by determining:
1) Potency vs HIV as determined by an EC50 in an initial pseudotype assay as described in the biology section.
2) Lack of general cytotoxicity vs a Hela cell line. >100uM was used as an arbitrary cut off for safety.
3) Measurement of the rate of metabolism vs human liver microsomal preparations and from this data projecting human rate of clearance. Lower is better.
4) Estimating potential exposure in man by measuring parameters such as AUC and rate of clearance by oral and iv dosing in rats. High exposure and low clearance was desired.

Aazaindole oxoacetic piperazine amides have been disclosed in two series of patent applications. The first series discloses azaindole derivatives which have promising potential as antiviral agents (hereinafter called, reference 94) Wang, Tao et al, U.S. patent 6476034 and WO 0162255 A1, filed January 19, 2001, published August 30, 2001. The second series (hereinafter called, reference 106) Wang, Tao, et al discloses HIV Antiviral Activity of Substituted Azaindoleoxoacetic Piperazine Derivatives in U.S. Patent Application Serial Number 10/214,982 filed August 7, 2002, which is a continuation-in-part application of U.S. Serial Number 10/038,306 filed January 2, 2002 (corresponding to PCT Int. Appl. (PCT/US02/00455), WO 02/062423 A1, filed January 2, 2002, published August 15, 2002. All of the references for these two series are incorporated by reference herein. Reference 106 describes in part C-7 heteroaryl, aryl or substituted 4,5,6,or 7-azaindoles as antiviral agents and is the most relevant prior art.

We have evaluated the properties of many compounds covered within the scope of references 94 and 106 and have found that the compounds having C-7, N-linked triazole groups are surprisingly and unexpectedly superior.

We initially evaluated compounds to determine which showed maximum potency or the lowest EC50 using the pseudotype assay described in the biology section of this application. In our case compounds with EC50s less than 0.20 nM were considered of most interest since this covered the most potent compounds and accounted for assay variability of our initial screen. The stability of compounds were also evaluated to determine metabolic stability when incubated in *in vitro* preparations of human liver microsomes (HLM). This is one commonly used predictive system for evaluating the potential for human metabolism and projecting clearance rates in man. Compounds with low clearance rates were most desireable. Intermediate and high clearance compounds would be more likely to have difficulty achieving feasible dosing regimen's in man vs low clearance compounds. Compounds for which accurate determinations could not be made were also not advanced.

Surprisingly, when the most promising compounds from the potency and metabolic stability criterias were evaluated in rats to measure their pharmacokinetic properties, one class of C-7 substituents, N linked triazoles of the invention showed very low clearance and very high AUCs (exposure) when compared to the compounds of references 94 and 106.

The C-7 N-linked triazoles of the invention thus showed surprising properties as they were essentially equivalent in potency to the most potent compounds covered by references 94 and 106 that we have evaluated to date. They showed metabolic stability in human liver microsomes that was equivalent to the best compounds from the application. Unexpectedly, they showed clearance rates in rats that were much lower, usually 10 fold lower than the best compounds from those described in the applications of reference 94 and were the best of any compounds evaluated in reference 106. Even more surprisingly, they were the only class of compounds to show significantly increased exposure in rats as shown by their AUCs.

In summation, these N-linked triazoles of the invention exhibited a surprising combination of properties that would not be obvious to one skilled in the art relying on the disclosure of references 94 and 106. Only a single triazole is disclosed in WO 02/06423. This compound has an R⁴ substiuent which is a C-linked triazole, and not an N-linked triazole, and exhibited potency which was not comparable to the N-linked triazoles of the invention No N-linked triazoles were described in the examples of the published PCT application from reference 106.

The following data tables summarize the potency, predicted human clearance based on human liver microsomes, and the AUC and clearance determined by pharmacokinetic studies in rats for these N-linked triazoles of the invention herein compared with representative compounds and close analogs contained in PCT application WO 02/062423 A1, filed January 2, 2002, published August 15, 2002 and the published applications and patents contained in reference 94. As seen in the following tables the N linked triazoles herein identified as most preferred groups exhibit surprising superiority especially in terms of displaying maximum potency, metabolic stability equivalent to best in class and uniquely a high AUC (exposure) and low clearance in rats which is determined by oral and iv dosing at 5mg/kg and 1mg/kg respectively. The rat model is an initial model used to assess potential for exposure in man.

The utility of compounds in the triazole class is surprisingly very dependent on the substitution patterns as depicted. For example the 1,2,3 triazoles attached at the 2-position nitrogen atom have to date shown significantly reduced AUC (exposure) in rats compared to the compounds depicted. In addition, moving the E group when E is methyl, in the 1,2,4-N-linked triazole from position 3 to 5 provides compounds with significantly reduced potency. As can be seen in Table A2, the N-linked triazoles specified showed high potency in an initial antiviral assay.

As evidenced by Tables A3-A5 of Comparator compounds, the metabolic stability of the N-linked triazole compounds Ia of the invention is surprisingly equivalent to or better than any of the compounds covered in either series of published azaindole applications (i.e. references 94 and 106).

As dramatically shown in the tables, the low clearance and high exposure seen in rats for the compounds in table A2 was surprising and unexpected since the prior art taught compounds did not exhibit these properties as one would have expected.

In the tables that follow these terms have the following in meanings:
"NT" meant not tested.
"Difficulties" means results could not be interpreted (i.e. in HLM test).

### Results

**Table A1. Biological Data Key for EC₅₀s in Tables A2-A7**

| Compounds* with ECs >1µM | Compounds with ECs >0.2 nM but <1µM | Compounds with EC50 ≤ 0.20 nM |
|---|---|---|
| Group 3 | Group 2 | Group 1 |

**Table A2 N linked Triazoles as R4 with Surprising Superior Properties**

| Example Number | EC50 category | CC50 >100uM | HLM predicted human clearance class | AUC 24h (ug.hr/mL) | CL, iv (mL/min/kg) |
|---|---|---|---|---|---|
| 1 | 1 | Yes | Low | 32 ± 12 | 1.6 ± 0.2 |
| 2 | 1 | Yes | Low | 52 ± 12 | 1.3 ± 0.19 |

**Table A3 A Relevant N linked Heteroaryl at R4 as Comparator**

| Example Number | EC50 category | CC50 > 100 µM | HLM predicted Human clearance class | AUC 24h (ug.hr/mL) | CL, iv (mL/min/kg) |
|---|---|---|---|---|---|
| 139 | 2 | Yes | High | | |

**Table A4 Some Relevant C linked Heteroaryl Comparators**

| Example Number | EC50 category | CC50 > 100 µM | HLM predicted Human clearance class | AUC 24h (ug.hr/mL) | CL, iv (mL/min/kg) |
|---|---|---|---|---|---|
| 40 | 2 | No | Low | | |
| 42 | 2 | Yes | Low | | |
| 22 | 1 | No | Intermediate | | |
| 35 | 1 | Yes | Intermediate | | |
| 37 | 1 | No | NT | | |
| 38 | 1 | No | Low | | |
| 39 | 2 | No | Intermediate | | |
| 28 | 3 | No | NT | | |
| 29 | 2 | No | High | | |
| 22 | 2 | No | High | | |
| 146 | 2 | Yes | Intermediate | | |

**Table A5 Some Relevant Comparators and data from U.S. patent 6476034 (Reference 94)**

| Reference Compound Number | EC50 category | CC50 >100uM? | HLM predicted human clearance class | AUC 24h (ug.hr/mL) | CL, iv (mL/min/kg) |
|---|---|---|---|---|---|
| 1 | 2 | Yes | Low | 0.5 | 32 ± 1.8 |
| 2 | 2 | Yes | Intermediate | | |
| 3 | 2 | Yes | Low | 6.3 ± 2.7 | 13±4.0 |
| 4 | 2 | Yes | Low | | |
| 5 | 1 | Yes | Intermediate | | |
| 6 | 2 | Yes | Low | 1.7 ± 0.58 | 31 ± 5.9 |
| 7 | 1 | Yes | Low | 2.6±0.12* | 19.3 ± 0.65 |
| 8 | 1 | Yes | Low | 1.03±0.07 | 47.2 ± 11.5 |
| 9 | 2 | Yes | Low | 5.9 ± 2.2 | 5.9 ± 2.2 |
| 10 | 2 | Yes | Low | 2.9 ± 0.3 | 11.7 ± 1.0 |
| 11 | 1 | Yes | | 1.4 ± 0.2* | 24.8 ± 0.41 |
| 12 | 1 | Yes | Low | 4.7 ± 1.1 | 11.9 ± 1.8 |
| 13 | 2 | Yes | NT | | |

### Structures of Reference Compounds as a Key for Table A5

### Reference Compound 1

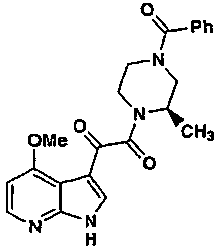

**Table A6 (Reference compounds structures 2-9)**

| | | | | | |
|---|---|---|---|---|---|
| 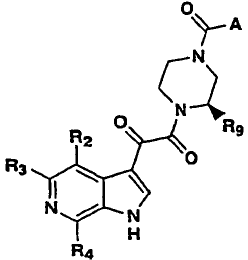 | | | | | |

| | | | | | |
|---|---|---|---|---|---|
| Reference Compound Number | R2 | R3 | R4 | R9 | A |
| 2 | OMe | H | X₂-OMe | (R)-Me | 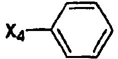 |
| 3 | OMe | H | X₂-OMe | H | 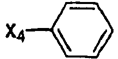 |
| 4 | OMe | H | X₂-OH | H | 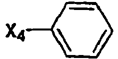 |
| 5 | Cl | H | 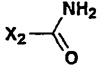 | H | 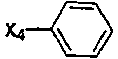 |
| 6 | F | H | 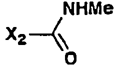 | H | 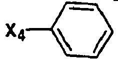 |
| 7 | F | H | 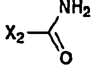 | H | 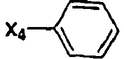 |
| 8 | MeO | H | 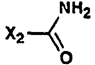 | H | 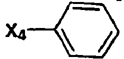 |
| 9 | MeO | H | 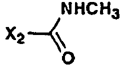 | H | 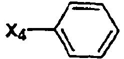 |

**Table A7 (Reference compounds 10-12)**

| | | | | | |
|---|---|---|---|---|---|
| 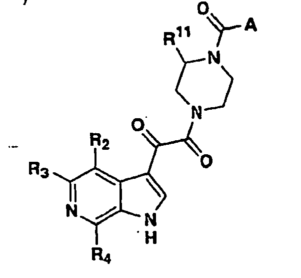 | | | | | |

| | | | | | |
|---|---|---|---|---|---|
| Reference Compound Number | R2 | R3 | R4 | R11 | A |
| 10 | MeO | H | X₂-OMe | (R)-Me | 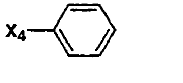 |
| 11 | MeO | H | 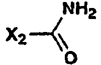 | (R)-Me | 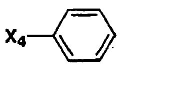 |
| 12 | MeO | H | 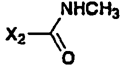 | (R)-Me | 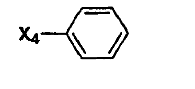 |

In Tables A6 and A7, X₂ and X₄ refer to point of attachment.

The compounds referred to in tables A3-A7 are disclosed and prepared in WO 02/062 423.

## Claims

1. A compound, including pharmaceutically acceptable salts thereof,

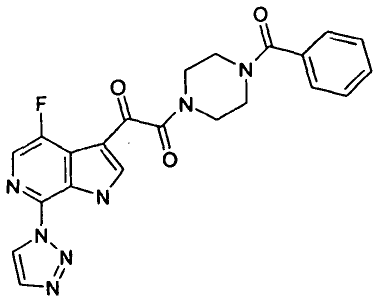

2. A compound, including Pharmaceutical acceptable salts thereof,

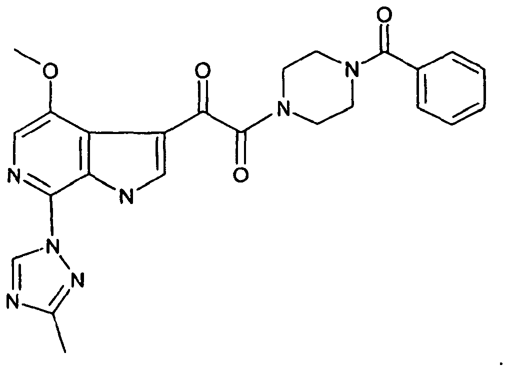

3. A pharmaceutical formulation which comprises an antiviral effective amount of a compound of Claim 1, and a pharmaceutically acceptable carrier.

4. A pharmaceutical formulation which comprises an antiviral effective amount of a compound of Claim 2, and a pharmaceutically acceptable carrier.

5. The pharmaceutical formulation of claim 3 or 4 which additionally comprises an antiviral effective amount of an AIDS treatment agent selected from the group consisting of:
(a) an AIDS antiviral agent;
(b) an anti-infective agent;
(c) an immunomodulator; and
(d) HIV entry inhibitors.

6. The compound of Claim 1 for use in treating mammals infected with the HIV virus.

7. The compound of Claim 2 for use in treating mammals infected with the HIV virus.

8. The compound of Claim 6 or 7 for use in treating mammals infected with the HIV virus additionally comprising the use of an antiviral effective amount of an AIDS treatment agent selected from the group consisting of:
(a) an AIDS antiviral agent;
(b) an anti-infective agent;
(c) an immunomodulator; and
(d) HIV entry inhibitors.

## Patentansprüche

1. Verbindung, einschließlich pharmazeutisch verträglicher Salze davon:

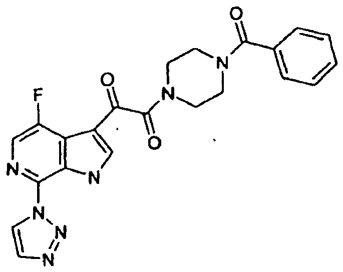

2. Verbindung, einschließlich pharmazeutisch verträglicher Salze davon:

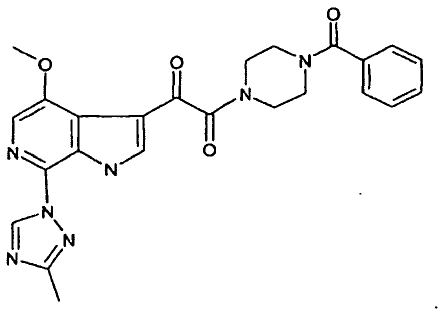

3. Pharmazeutische Formulierung, die eine antiviral wirksame Menge einer Verbindung nach Anspruch 1 und einen pharmazeutisch verträglichen Träger umfasst.

4. Pharmazeutische Formulierung, die eine antiviral wirksame Menge einer Verbindung nach Anspruch 2 und einen pharmazeutisch verträglichen Träger umfasst.

5. Pharmazeutische Formulierung nach Anspruch 3 oder 4, die zusätzlich eine antiviral wirksame Menge eines Mittels zur Behandlung von AIDS umfasst, das aus er Gruppe ausgewählt ist, bestehend aus:
(a) einem antiviralen Mittel gegen AIDS;
(b) einem antiinfektiösen Mittel;
(c) einem Immunmodulator; und
(d) HIV-Eintrittsinhibitoren.

6. Verbindung nach Anspruch 1 zur Verwendung in der Behandlung von mit dem HIV-Virus infizierten Säugern.

7. Verbindung nach Anspruch 2 zur Verwendung in der Behandlung von mit dem HIV-Virus infizierten Säugern.

8. Verbindung nach Anspruch 6 oder 7 zur Verwendung in der Behandlung von mit dem HIV-Virus infizierten Säugern, die zusätzlich die Verwendung einer antiviral wirksamen Menge eines Mittels zur Behandlung von AIDS umfasst, das aus der Gruppe ausgewählt ist, bestehend aus:
(a) einem antiviralen Mittel gegen AIDS;
(b) einem antiinfektiösen Mittel;
(c) einem Immunmodulator; und
(d) HIV-Eintrittsinhibitoren.

## Revendications

1. Composé, y compris des sels pharmaceutiquement acceptables de celui-ci,

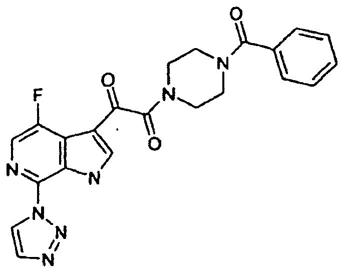

2. Composé, y compris des sels pharmaceutiquement acceptables de celui-ci,

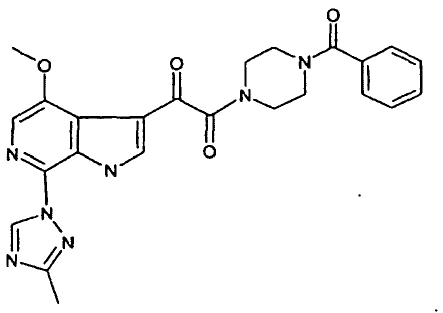

3. Formulation pharmaceutique qui comprend une quantité antivirale efficace d'un composé selon la revendication 1 et un véhicule pharmaceutiquement acceptable.

4. Formulation pharmaceutique qui comprend une quantité antivirale efficace d'un composé selon la revendication 2 et un véhicule pharmaceutiquement acceptable.

5. Formulation pharmaceutique selon la revendication 3 ou 4, qui comprend en plus une quantité antivirale efficace d'un agent de traitement du SIDA sélectionné parmi le groupe consistant en:
(a) un agent antiviral contre le SIDA;
(b) un agent anti-infectieux;
(c) un immunomodulateur et
(d) des inhibiteurs d'entrée du VIH.

6. Composé selon la revendication 1, à utiliser dans le traitement des mammifères infectés par le virus VIH.

7. Composé selon la revendication 2, à utiliser dans le traitement des mammifères infectés par le virus VIH.

8. Composé selon la revendication 6 ou 7, à utiliser dans le traitement des mammifères infectés par le virus VIH, comprenant en plus l'utilisation d'une quantité antivirale efficace d'un agent de traitement du SIDA sélectionné parmi le groupe consistant en:
(a) un agent antiviral contre le SIDA;
(b) un agent anti-infectieux;
(c) un immunomodulateur et
(d) des inhibiteurs d'entrée du VIH.
